# EUROPEAN PATENT SPECIFICATION

(11) **EP 1 877 097 B1**
(45) Date of publication and mention of the grant of the patent: **20.06.2012**
(21) Application number: 05785192.5
(22) Date of filing: 11.08.2005
(51) Int. Cl.: A61K 47/48, C07D 311/92, A61P 35/00

(54) **AMINOACID CONJUGATES OF BETA-LAPACHONE FOR TUMOR TARGETING**
KONJUGATE AUS BETA-LAPACHON UND AMINOSÄUREN FÜR TUMOR-TARGETING
CONJUGUÉS D'ACIDES AMINÉS ET DE BÊTA-LAPACHONE POUR LE CIBLAGE DE TUMEURS

(30) Priority: 11.08.2004 US 600373 P
(43) Date of publication of application: 16.01.2008
(73) Proprietor: ARQULE, INC., Woburn, MA 01801-5140 (US)
(72) Inventor: ASHWELL, Mark, Carlisle, MA, 01741 (US); TANDON, Manish, Framingham, Massachusetts 01701 (US); LAPIERRE, Jean-Marc, Pelham, New Hampshire 03076 (US); LIU, Yanbin, Acton, Massachusetts 01720 (US)
(74) Representative: Crump, Julian Richard John
(86) International application number: PCT/US2005/028438
(87) International publication number: WO 2006/020719

(56) References cited:
- WO-A-02/058694
- WO-A-03/011224
- WO-A-03/090710
- WO-A-2005/082356
- WO-A-2005/082357
- WO-A-2006/020722
- DI CHENNA P. H. ET AL: "Preparation and cytotoxicity toward cancer cells of mono(arylimino) derivatives of b-lapachone" JOURNAL OF MEDICINAL CHEMISTRY, AMERICAN CHEMICAL SOCIETY, US, vol. 44, 2001, pages 2486-2489, XP008068680 ISSN: 0022-2623
- POWIS G ET AL: "MOLECULAR PHARMACOLOGY AND ANTITUMOR ACTIVITY OF PALMARUMYCIN-BASED INHIBITORS OF THIOREDOXIN REDUCTASE" MOLECULAR CANCER THERAPEUTICS, AMERICAN ASSOCIATION OF CANCER RESEARCH, US, vol. 5, no. 3, 2006, pages 630-636, XP008071878 ISSN: 1535-7163
- KUMI-DIAKA JAMES ET AL: "Potential mechanism of phytochemical-induced apoptosis in human prostate adenocarcinoma cells: Therapeutic synergy in genistein and [beta]-lapachone combination treatment" CANCER CELL INTERNATIONAL, BIOMED CENTRAL, LONDON, GB, vol. 4, no. 1, 17 August 2004 (2004-08-17), page 5, XP021007276 ISSN: 1475-2867
- REINICKE K E ET AL: "DEVELOPMENT OF BETA-LAPACHONE PRODRUGS FOR THERAPY AGAINST HUMAN CANCER CELLS WITH ELEVATED NAD(P)H:QUINONE OXIDOREDUCTASE 1 LEVELS" CLINICAL CANCER RESEARCH, THE ASSOCIATION, DENVILLE, NJ, US, vol. 11, no. 8, 15 April 2005 (2005-04-15), pages 3055-3064, XP008046994 ISSN: 1078-0432

## Description

### FIELD OF THE INTENTION

The present invention relates to improved therapeutic agents for treating cancer. More particularly, the invention relates to improved quinone and β-lapachone compositions according to the claims for the treatment of cancer.

### BACKGROUND OF THE INVENTION

The quinones are a large and varied group of natural products found in all major groups of organisms. Quinones are a group of aromatic dioxo compounds derived from benzene or multiple-ring hydrocarbons such as naphthalene, anthracene, *etc.* They are classified as benzoquinones, naphthoquinones, anthraquinones, *etc:,* on the basis of the ring system. Quinones have a variety of medicinal and industrial uses.

Many efficient antineoplastic drugs are either quinones (anthracycline derivatives, mitoxantrone, actinomycin), quinonoid derivatives (quinolones, genistein, bactracyclin), or drugs such as etoposide that can easily be converted to quinones by *in vivo* oxidation. Gantchev et al. (1997) Biochem. Biophys. Res. Comm. 237:24-27. Quinones are now widely used as anticancer, antibacterial and anti-malarial drugs, as well as fungicides. The antitumor activities of the quinones were revealed more than two decades ago when the National Cancer Institute published a report in which fifteen-hundred synthetic and natural quinones were screened for their anticancer activities. Driscoll et al. (1.974) Cancer Cheroot. Reports 4:1-362.

More particularly, β-lapachone (3,4-dihydro-2,2-dimethyl-2H-naphtho[1,2-b]pyran-5,6-dione), a quinone, is derived from lapachol (a naphthoquinone) which can be isolated from the lapacho tree (*Tabebuia avellanedae*), a member of the catalpa family (*Bignoniaceae*). Lapachol and β-lapachone (with numbering) have the following chemical structures:

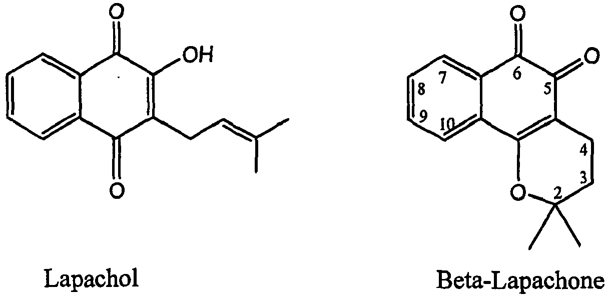

β-lapachone, as well as its intermediates, derivatives and analogs thereof, are described in Li, C.J. et al., (1993) J. Biol. Chem., 268(30): 22463-22468.

As a single agent, β-lapachone has demonstrated significant antineoplastic activity against human cancer cell lines at concentrations typically in the range of 1-10 µM (IC₅₀). Cytotoxicity has been demonstrated in transformed cell lines derived from patients with promyelocytic leukemia (Planchon et al., (1996) Cancer Res., 55: 3706-3711), prostate (Li, C.J., et al., (1995) Cancer Res., 55: 3712-3715), malignant glioma (Weller, M. et al., (1997) Int. J. Cancer, 73: 707-714), hepatoma (Lai, C.C., et al., (1998) Histol Histopathol, 13: 89-97), colon (Huang, L., et al., (1999) Mol Med, 5,: 711-720), breast (Wuertzberger, S.M., et al., (1998) Cancer Res., 58: 1876), ovarian (Li, C.J. et al., (1999) Proc. Natl. Acad. Sci. USA, 96(23): 13369-13374), pancreatic (Li, Y., et al., (2000) Mol Med, 6: 1008-1015; Li, Y., (1999) Mol Med, 5: 232-239), and multiple myeloma cell lines, including drug-resistant lines (Li, Y., (2000) Mol Med, 6: 1008-1015). No cytotoxic effects were observed on normal fresh or proliferating human PBMC (Li, Y., (2000) Mol Med, 6: 1008-1015).

j3-lapachone appears to work by inducing unscheduled expression of checkpoint molecules, *e.g*., E2F, independent of DNA damage and cell cycle stages. Several studies have shown that β-lapachone activates checkpoints and induces apoptosis in cancer cells from a variety of tissues without affecting normal cells from these tissues (U.S. Patent Application Publication No. 2002/0169135). In normal cells with their intact regulatory mechanisms, such an imposed expression of a checkpoint molecule results in a transient expression pattern and causes little consequence. In contrast, cancer and pre-cancer cells have defective mechanisms, which result in unchecked and persistent expression of unscheduled checkpoint molecules, *e.g*., E2F, leading to selective cell death in cancer and pre-cancer cells.

β-lapachone has been shown to be a DNA repair inhibitor that sensitizes cells to DNA-damaging agents including radiation (Boothman, D. A. et al., Cancer Res, 47 (1987) 5361; Boorstein, R. J., et al., Biochem. Biophys. Commun., 117 (1983) 30). β-lapachone has also shown potent *in vitro* inhibition of human DNA Topoisomerases I (Li, C. J. et al., J. Biol. Chem., 268 (1993) 22463) and II (Frydman, B. et al., Cancer Res., 57 (1997) 620) with novel mechanisms of action. Unlike topoisomerase "poisons" (*e.g*., camptothecin, etoposide, doxorubicin) which stabilize the covalent topoisomerase-DNA complex and induce topoisomerase-mediated DNA cleavage, β-lapachone interacts directly with the enzyme to inhibit catalysis and block the formation of cleavable complex (Li, C. J. et al., J. Biol. Chem., 268 (1993) 22463) or with the complex itself, causing religation of DNA breaks and dissociation of the enzyme from DNA (Krishnan, P. et al., Biochem Pharm, 60 (2000) 1367). β-lapachone and its derivatives have also been synthesized and tested as anti-viral and anti-parasitic agents (Goncalves, A. M., et al., Mol. Biochem. Parasitology, 1 (1980) 167-176; Schaffner-Sabba, K., et al., J. Med. Chem., 27 (1984) 990-994).

More specifically, β-lapachone appears to work by disrupting DNA replication, causing cell-cycle delays in G1 and/or S phase, inducing either apoptotic or necrotic cell death in a wide variety of human carcinoma cell lines without DNA damage and independent of p53 status (Li, Y. Z*. et al.* (1999); Huang, L. *et al*.). Topoisomerase I is an enzyme that unwinds the DNA that makes up the chromosomes. The chromosomes must be unwound in order for the cell to use the genetic information to synthesize proteins; β-lapachone keeps the chromosomes wound tight, so that the cell cannot make proteins. As a result, the cell stops growing. Because cancer cells are constantly replicating and circumvent many mechanisms that restrict replication in normal cells, they are more vulnerable to topoisomerase inhibition than are normal cells.

Another possible intracellular target for β-lapachone in tumor cells is the enzyme NAP(P)H:quinone oxidoreductase (NQO1). Biochemical studies suggest that reduction of β-lapachone by NQO1 leads to a "futile cycling" between the quinone and hydroquinone forms with a concomitant loss of reduced NADH or NAD(P)H (Pink, J. J. et al., J Biol Chem., 275 (2000) 5416). The exhaustion of these reduced enzyme cofactors may be a critical factor for the activation of the apoptotic pathway after β-lapachone treatment.

As a result of these findings, β-lapachone is actively being developed for the treatment of cancer and tumors. In WO 00/61142, for example, there is disclosed a method and composition for the treatment of cancer, which comprises the administration of an effective amount of a first compound, a G₁ or S phase drug, such as a β-lapachone, in combination with a G₂/M drug, such as a taxane derivative. Additionally, U.S. Pat. No. 6,245,807 discloses the use of β-lapachone, amongst other β-lapachone derivatives, for use in the treatment of human prostate disease.

In addition to β-lapachone, a number of β-lapachone analogs having antiproliferative properties have been disclosed in the art, such as those described in PCT International Application PCT/US93/07878 (WO 94/04145), and U.S. Pat. No. 6,245,807, in which a variety of substituents may be attached at positions 3- and 4- on the β-lapachone compound. PCT International Application PCT/US00/10169 (WO 00/61142), discloses β-lapachone, which may have a variety of substituents at the 3-position as well as in place of the methyl groups attached at the 2-position. U.S. Patent Nos. 5,763,625, 5,824,700, and 5,969,163, disclose analogs with a variety of substituents at the 2-, 3- and 4-positions. Furthermore, a number of journals report β-lapachone analogs with substituents at one or more of the following positions: 2-, 3-, 8- and/or 9-positions, (See, Sabba et al. , (1984) J Med Chem 27:990-994 (substituents at the 2-, 8-and 9- positions); (Portela and Stoppani, (1996) Biochem Pharm 51:275-283 (substituents at the 2- and 9-positions); Goncalves et al., (1998) Molecular and Biochemical Parasitology 1:167-176 (substituents at the 2- and 3- positions)).

Moreover, structures having sulfur-containing hetero-rings in the "a" and "β" positions of lapachone have been reported (Kurokawa S, (1970) Bulletin of The Chemical Society of Japan 43:1454-1459; Tapia, RA et al., (2000) Heterocycles 53(3):585-598; Tapia, RA et al., (1997) Tetrahedron Letters 38(1):153-154; Chuang, CP et al., (1996) Heterocycles 40(10):2215-2221; Suginome H et al., (1993) Journal of the Chemical Society, Chemical Communications 9:807-809; Tonholo J et al., (1988) Journal of the Brazilian Chemical Society 9(2):163-169; and Krapcho AP et al., (1990) Journal of Medicinal Chemistry 33(9):2651-2655). More particularly, hetero β-lapachone analogs are disclosed in PCT International Application PCT/US031037219 (WO 04/045557).

Quinones also have a number of other medicinal uses. Terpenoid-type quinones are also useful as treatments for diabetes, (U.S. Pat. No. 5,674,900).Additional quinones can be used to treat cirrhosis and other liver disorders, (U.S. Pat. Nos. 5,210,239 and 5,385,942).

Hydroquinone amines and quinone amines are also useful for treating a number of conditions, including spinal trauma and head injury (U.S. Pat. No. 5,120,843). Degenerative central nervous system diseases, as well as vascular diseases, are treatable with quinones such as Idebenone [2,3-dimethoxy-5-methyl-6-(10-hydroxydecyl)-1,4-benzoquinone] and Rifamycin. S. Mordente et al. (1998) Chem. Res. Toxicol. 11:54-63; Rao et al. (1997) Free Radic. Biol. Med 22:439-46; Cortelli et al. (1997) J. Neurol. Sci. 148:25-31; and Mahadik et al. (1996) Prostaglandins Leukot. Essent. Fatty Acids 55:45-54. A vitamin K analog, 6-cyclo-octylamino-5,8-quinoline quinone shows efficacy for treatment of leprosy and tuberculosis (U.S. Pat. No. 4,963,565). Hydroquinone is also used to treat skin pigmentation disorders. Clarys et al. (1998) J. Dermatol. 25:412-4. Mitomycin C-related drug indoloquinone EO9 has demonstrated cell killing against HL-60 human leukemia cells, H661 human lung cancer cells, rat Walker tumor cells and human HT29 colon carcinoma cells. Begleiter et al. (1997) Oncol. Res. 9:371-82; and Bailey et al. (1997) Br. J. Cancer 76:1596-603.

Quinones such as aloin, a C-glycoside derivative of anthraquinone, accelerate ethanol oxidation and may be useful in treating acute alcohol intoxication. Chung et al. (1996) Biochem. Pharmacol. 52:1461-8 and Nanji et al. (1996) Toxicol. Appl. Pharmacol. 140:101-7. Quinones capsaicin and resiniferatoxin blocked activation of nuclear transcription factor NF-κB, which is required for viral replication, immune regulation and induction of various inflammatory and growth-regulatory genes. Singh et al. (1996) J. Immunol. 157:4412-20. Antiretroviral and antiprotozoan naphthoquinones are described in U.S. Pat. Nos. 5,780,514 and 5,783,598. Anthraquinones are also useful as laxatives. Ashraf et al. (1994) Aliment. Pharmaco/. Ther. 8:329-36; and Muller-Lissner (1993) Pharmacol. 47 (Suppl. 1): 138-45.

Because of the wide variety of biological processes in which quinones play a critical role, it would be advantageous to develop novel quinones for various uses, including disease treatment.

One obstacle, however, to the development of pharmaceutical formulations comprising quinones, such as β-lapachone or β-lapachone analogs for pharmaceutical use is the low solubility of many quinone compounds, including β-lapachone compounds, in pharmaceutically acceptable solvents. There are also drawbacks related to the pharmacokinetic profiles of traditional formulations comprising quinones. As a result, there is a need for improved formulations of quinone compounds for pharmaceutical administration, which are both safe and readily bioavailable to the subject to which the formulation is administered.

### SUMMARY OF THE INVENTION

In accordance with the present invention there is provided a quinone prodrug composition comprising a quinone compound of formula Ia: 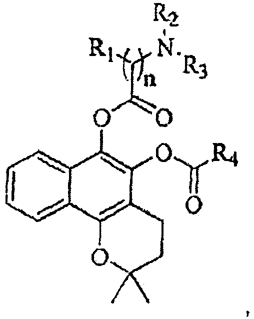 wherein:
R₁ is H, alkyl, alkyl substituted with a sulfyl (-SH or thio alkyl), or (C₁-C₄) alcohol, or together with R₂, forms a saturated, unsaturated, or aromatic cyclic moiety;
R₂ and R₃ are each independently H, alkyl, or aromatic, or together form a saturated, unsaturated, or aromatic cyclic moiety
R₄ is alkyl, the side chain of a naturally occurring alpha amino acid, or the side chain, alpha carbon, and the alpha amino group of a naturally occurring amino acid; and
n is 1, 2 or 3.

In another aspect of the present invention there is provided a pharmaceutical composition comprising a therapeutically effective amount of at least one quinone prodrug composition of the present invention and a pharmaceutically acceptable excipient.

There are herein described quinone prodrug compositions according to the claims comprising a quinone compound covalently linked to one or two pro-moieties, such as an amino acid moiety. The quinone compound may be released from the pro-moiety *via* hydrolytic, oxidative, and/or enzymatic release mechanisms.

The quinone prodrug compositions exhibit the added benefit of increased aqueous solubility, improved stability, and improved pharmacokinetic profiles. The pro-moiety may be selected to obtain desired prodrug characteristics. For example, the pro-moiety, *e.g*., an amino acid moiety, may be selected based on solubility, stability, bioavailability, and/or *in vivo* delivery or uptake.

There is also herein described therapeutic methods using the quinone prodrug compositions according to the claims. The methods can be used to treat or prevent any disease or condition in which the quinone compound is useful. In particular, the methods of the invention relate to the treatment of cancer.

There are also herein described, pharmaceutical compositions useful in the methods herein described. The pharmaceutical compositions may be formulated with pharmaceutically acceptable excipients such as carriers, solvents, stabilizers, adjuvants, diluents, *etc*., depending upon the particular mode of administration and dosage form.

These and other aspects of the invention are discussed in further detail below.

### CERTAIN EMBODIMENTS

1. A quinone prodrug composition comprising a quinone compound of formula Ia: 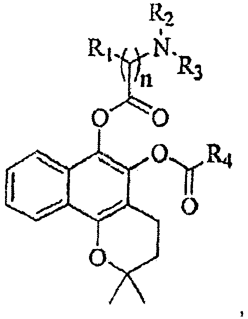 wherein:
   R₁ is H, alkyl, alkyl substituted with a sulfyl (-SH or thio alkyl), or (C₁-C₄) alcohol, or together with R₂, forms a saturate, unsaturated, or aromatic cyclic moiety;
   R₂ and R₃ are each independently H, alkyl, or aromatic, or together form a saturated, unsaturated, or aromatic cyclic moiety
   R₄ is alkyl, the side chain of a naturally occurring alpha amino acid, or the side chain, alpha carbon, and the alpha amino group of a naturally occurring amino acid; and
   n is 1, 2 or 3.
2. The quinone prodrug composition of embodiment 1, wherein the quinone compound is selected from the group consisting of: 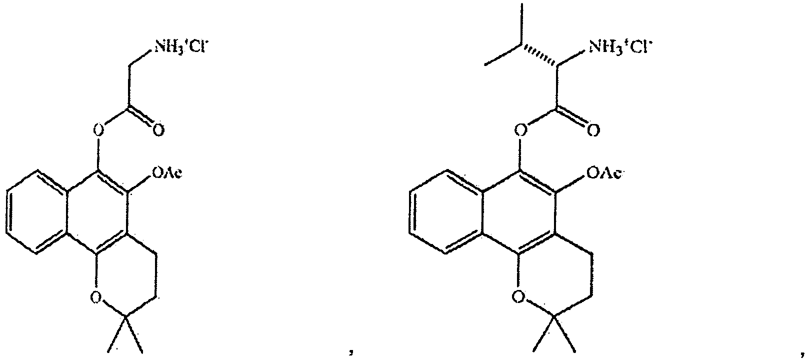 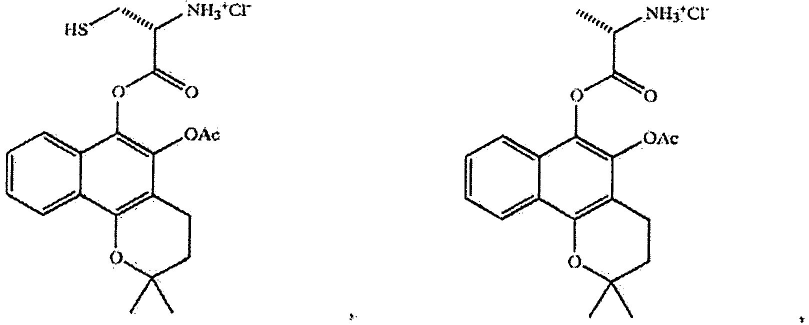 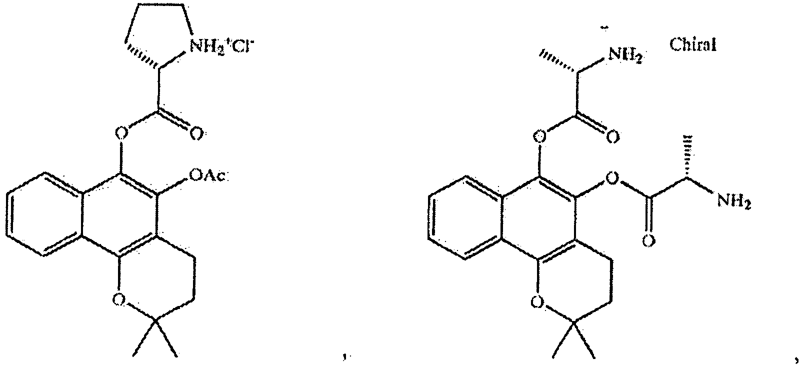 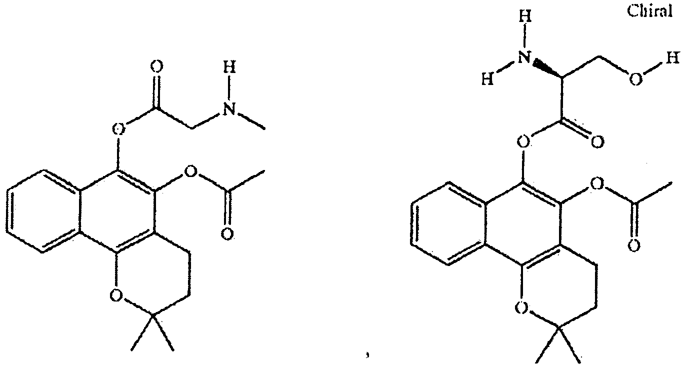 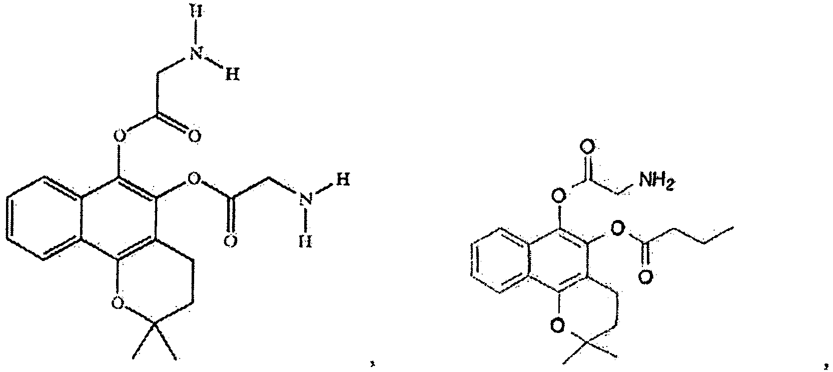 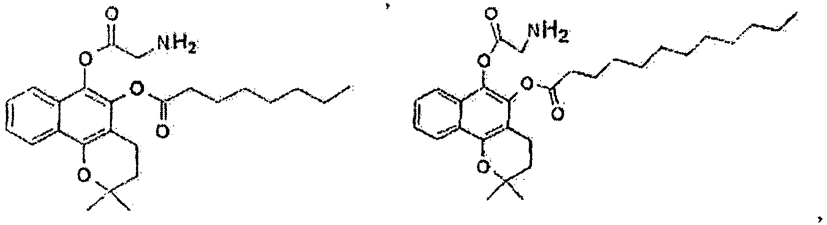 , and 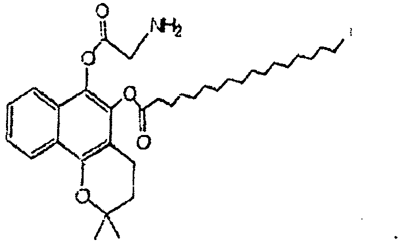
3. The quinone prodrug composition of embodiment 1, wherein the quinone compound is: 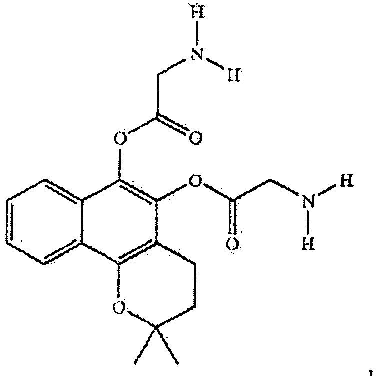 or a HCl salt, a mesylate salt or a tosylate salt thereof.
4. A pharmaceutical composition comprising a therapeutically effective amount of at least one quinone prodrug composition of embodiment 1 and a pharmaceutically acceptable excipient.
5. The pharmaceutical composition of embodiment 4, wherein the pharmaceutical composition is an aqueous solution.
6. The pharmaceutical composition of embodiment 4, wherein the pharmaceutical composition is a lyophilized solid.
7. The pharmaceutical composition of embodiment 4, wherein the pharmaceutical composition comprises 0.1 mg/ml to 10 mg/ml of the quinone prodrug composition.
8. The pharmaceutical composition of embodiment 4, further comprising a second anticancer agent.
9. The pharmaceutical composition of embodiment 8, wherein the second anticancer agent is selected from the group consisting of taxane derivatives, gemcitabine, nucleotide and nucleotide anticancer agents, cisplatin, imatnibmeasylate, and trastuzumab.
10. The pharmaceutical composition of embodiment 9. wherein the taxane derivative is paclitaxel or docetaxol.

### BRIEF DESCRIPTION OF THE DRAWINGS

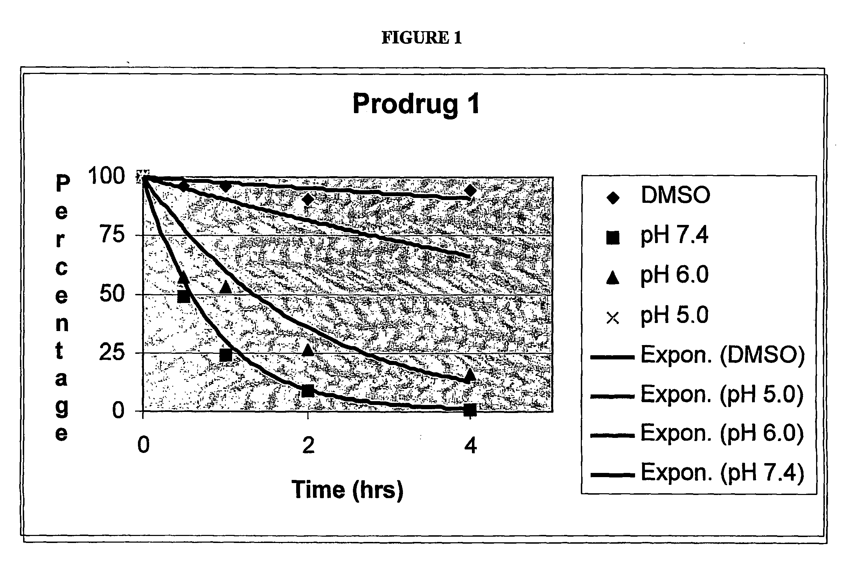

Figure 1 illustrates the release of β-lapachone form a preferred a β-lapachone prodrug composition of the invention.

### DETAILED DESCRIPTION OF THE INVENTION

There is herein described quinone prodrug compositions according to the claims comprising a quinone compound covalently linked to one or two pro-moieties, such as an amino acid moiety , to thereby result in a quinone prodrug composition. The quinone compound may be released from the pro-moiety via hydrolytic, oxidative, and/or enzymatic release mechanisms.

The quinone prodrug compositions according to the claims exhibit the added benefit of increased aqueous solubility, improved stability, and improved pharmacokinetic profiles. The pro-moiety may be selected to obtain desired prodrug characteristics. For example, the pro-moiety, *e.g.*, an amino acid moiety, may be selected based on solubility, stability, bioavailability, and/or *in vivo* delivery or uptake.

### A. Quinone and β-Lapachone Compounds

The prodrug compositions comprise a quinone compound according to the claims. As discussed above, β-lapachone has the following chemical structure (Compound 1, with numbering):

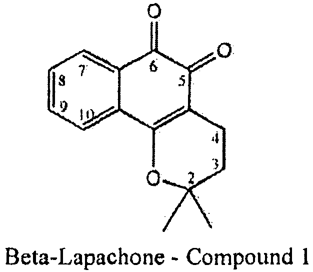

### B. Pro-Moieties

The pro-moiety herein described may be selected so as to obtain desired solubility, stability, bioavailability, pharmacokinetic properties, or targeted/selective *in vivo* drug delivery. The pro-moiety may be covalently attached to either or both of the quinone carbonyl groups.

The pro-moiety is an amino acid moiety.

Pro-moieties as herein described also include the side chain of a naturally occurring alpha amino acid or the side chain, alpha carbon, and the alpha amino group of a naturally occurring amino acid (e.g., an alpha amino acid without its carboxyl group).

Naturally occurring alpha amino acids include alanine, valine, leucine, isoleucine, phenylalanine, tryptophan, methionine, glycine, serine, threonine, cysteine, tyrosine, asparagine, glutamine, aspartic acid, glutamic acid, lysine, arginine, proline, and histidine. Preferred amino acid residues include glycine, alanine, valine and proline.

### C. Quinone Prodrug Compositions and β-Lapachone Prodrug Compositions

As discussed above, the quinone prodrug compositions according to the claims generally comprise a quinone compound covalently attached to one or two pro-moieties, such as an amino acid moiety.

Prodrugs of the present invention comprising an amino acid moiety are illustrated in Formula la below 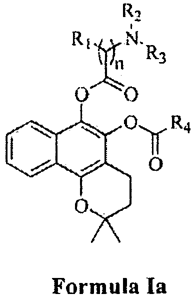 wherein
R₁ is independently selected from H; or alkyl, optionally substituted with a sulfyl (-SH or thio alkyl) group; or together with R₂ forms a saturated, unsaturated, or aromatic cyclic moiety;
R₂ and R₃ are independently H, alkyl, aromatic, or together form a saturated, unsaturated, or aromatic cyclic moiety;
R₄ is independently alkyl, aromatic, or can be the side chain of a naturally occuring alpha amino acid, or the side chain, alpha carbon, and the alpha amino group of a naturally occuring amino acid ;
n is 1, 2, or 3.

In a further embodiment, R₁ is a (C₁- C₄) alcohol and R₂, R₃, R₄ and n are as described above.

The moiety 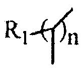 in Formula la bears an R₁ group on each of the n = 1 to n = 3 carbons atom in parentheses. That moiety also bears a hydrogen atom (not shown) on each of the n = 1 to n = 3 carbons atom in parentheses as necessary to form properly tetravalent carbon atoms. A carbon atom in parentheses linked by a double bond to another atom need not bear any additional hydrogen atoms to be properly tetravalent. For example, when R₁ and R₂ taken together form a cyclic aromatic moiety, the carbon atom in parentheses need not bear an additional hydrogen atom.

In an alternative embodiment of compounds of Formula Ia, when an independently selected R₁ group is hydrogen or alkyl, the carbon atom to which it is immediately bound may bear an independently selected hydrogen or methyl group. For example, the partial structure of the 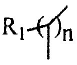 moiety from compounds of Formula la where n =2 can be represented as: - (C)R₁-C)R₁-, and may be -C(H)(CH₃)-C(H)(CH₃) - when both independently selected R₁ groups are methyl, and may be -C(CH₃)(CH₃)-C(CH₂CH₃)(CH₃)- when R₁ is methyl in one instance and ethyl in the other instance, and each carbon bears an additional methyl group. In another example, when n = 1, R₁ is methyl, and the carbon atom in parentheses bears an additional methyl group, then 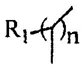 is -C(CH₃)₂-, as in examples 16 and 17, which exemplify the use of 2-methylalanine.

Certain preferred β-lapachone prodrug compositions include Prodrug 1 through Prodrug 28, as illustrated in the table below.

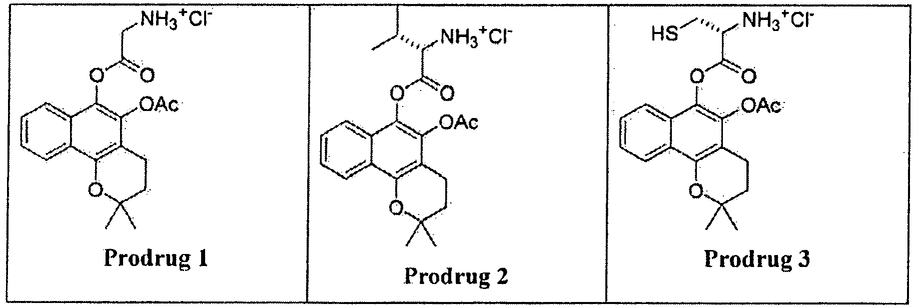

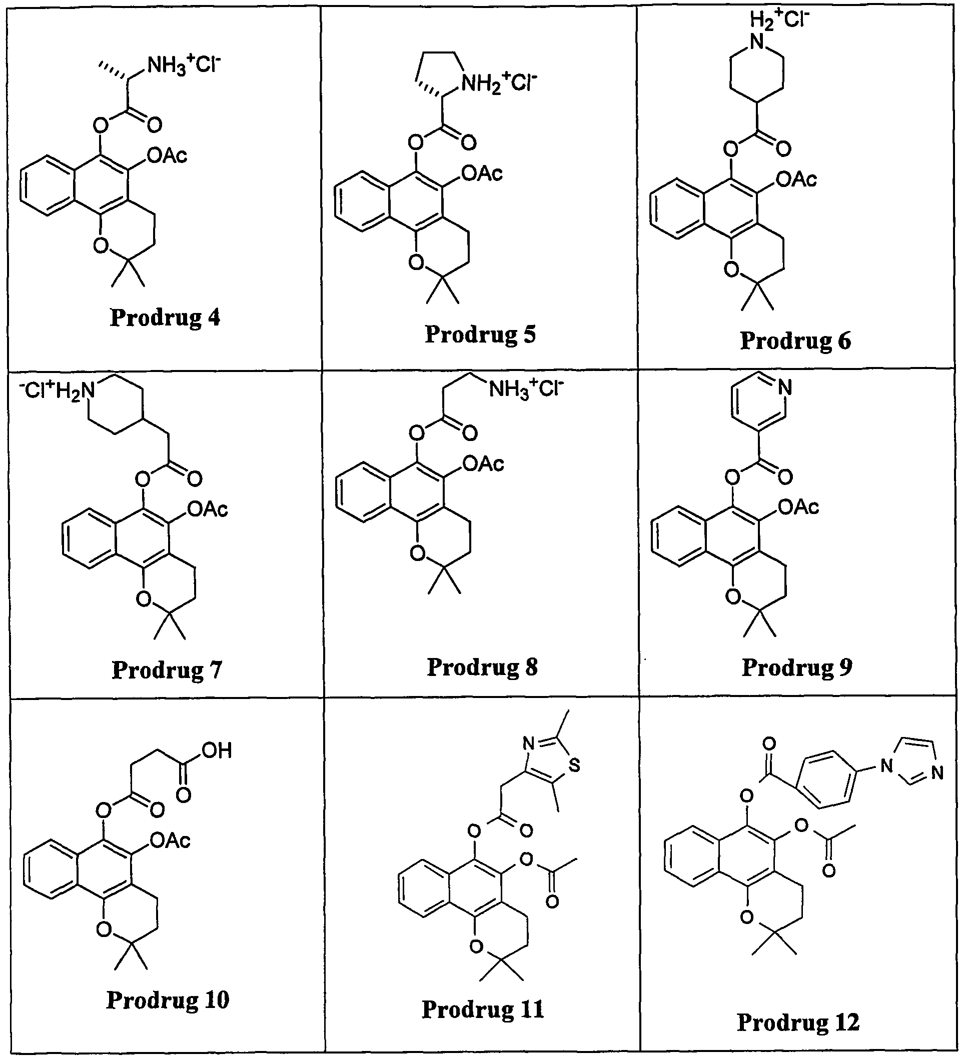

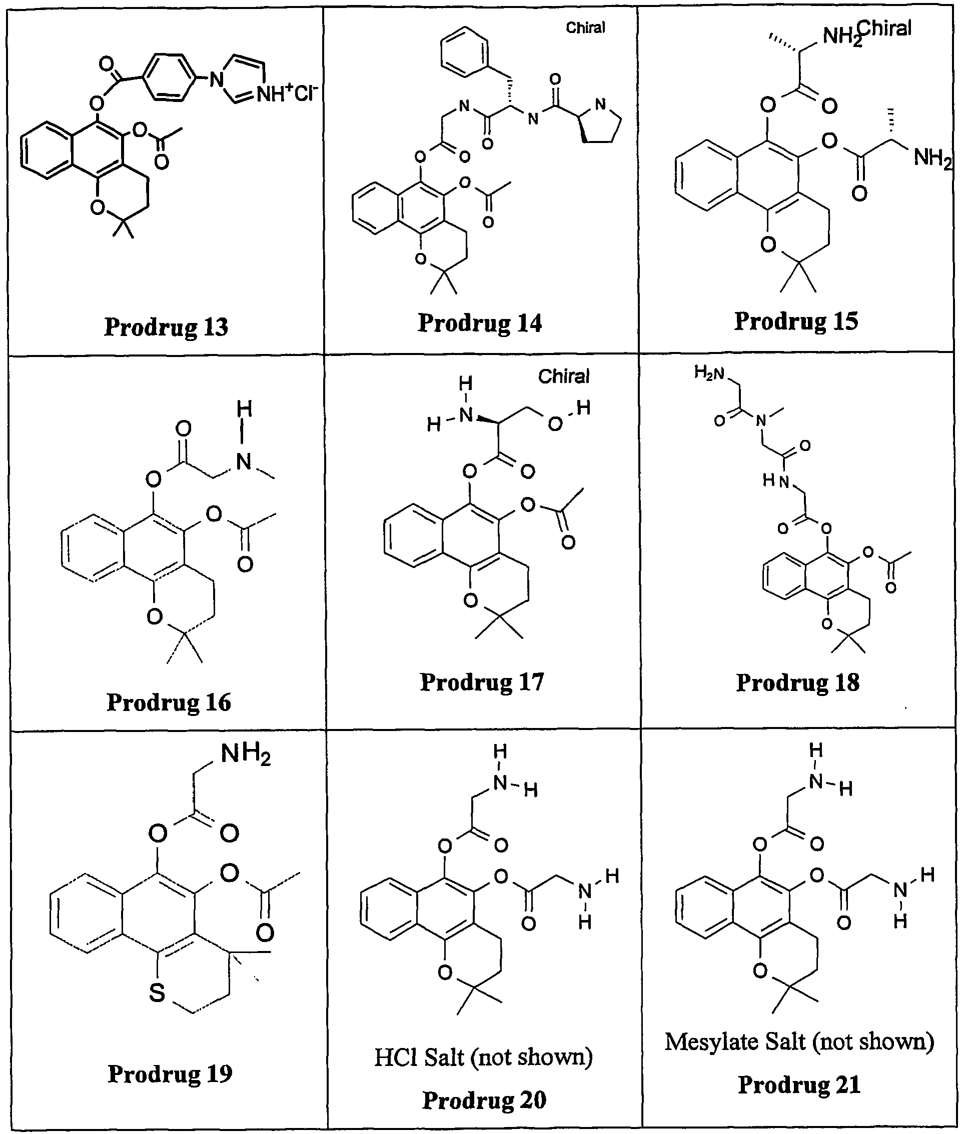

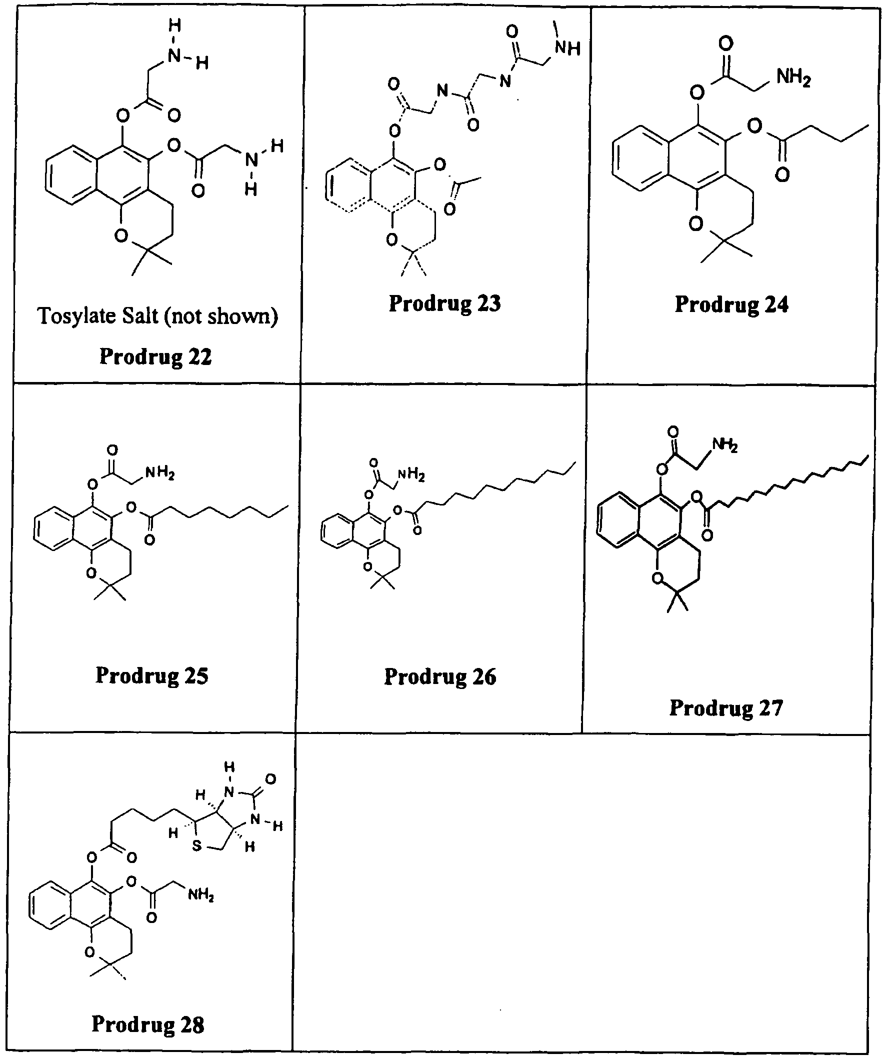

Other preferred prodrug compositions include, those described in the examples.

The quinone prodrug compositions herein described may be prepared in any manner known in the art. For example, the β-lapachone prodrug compositions of Formula In of the invention may be prepared in accordance with Scheme la, illustrate below. However, many modifications can be made to the synthesis scheme, as recognized by those skilled in the art.

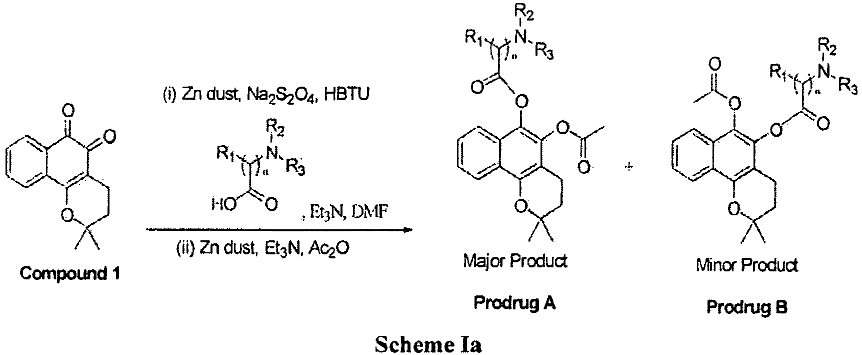

If substitution of the amino acid moiety at both quinone carbonyl groups is desired, two eqivalents of the amino acid moiety may be used under conditions similar to those depicted in Scheme Ia. Further, protecting groups may be used if desired under the reaction conditions.

### D. Methods

Also herein described are therapeutic methods. The methods can be used to treat or prevent any disease or condition in which the quinone compound is useful. In particular, the methods relate to the treatment of cancer.

More particularly, the methods herein described include methods for treating cancer comprising administering a composition comprising a therapeutically effective amount of at least one quinone prodrug composition herein described to a subject in need thereof.

Also herein described are methods for obtaining improved plasma half-life of quinone compounds *in vivo* comprising administering a therapeutically effective amount of at least one quinone prodrug composition herein described to a subject in need thereof, wherein the quinone prodrug exhibits an improved plasma half-life *in vivo* as compared to administration of the quinone compound not in a prodrug form.

The methods herein described are particularly useful for the treatment of mammalian cancers, including lung, breast, colon, ovarian and prostate cancers, multiple myeloma and malignant melanoma, or for improving the accumulation of drug in such cancer tissues.

According to the methods herein described, the compound(s) may be administered to the subject *via* any drug delivery route known in the art. Specific exemplary administration routes include peripheral and central routes such as oral, ocular, rectal, buccal, topical, nasal, ophthalmic, subcutaneous, intramuscular, intraveneous (bolus and infusion), intracerebral, transdermal, and pulmonary. The quinone prodrug compositions herein described are particularly suited for oral or parenteral administration.

The term "therapeutically effective amount", as used herein, refers to an amount of a pharmaceutical agent to treat, ameliorate, or prevent the identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician.

For any compound, the therapeutically effective amount can be estimated initially either in cell culture assays, *e.g*., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, *e.g*., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, ED₅₀/LD₅₀. Pharmaceutical compositions that exhibit large therapeutic indices are preferred. The dosage may vary within this range depending upon the dosage form employed, sensitivity of the patient, and the route of administration.

The compounds herein described may be administered at doses that vary from 0. 1 µg to 100,000 mg, depending upon the route of administration. The preferred dose will be in the range of about 0.1 mg/day to about 10 g/day, or more preferably about 0.1 mg to about 3g/day, or still more about 0.1 mg to about 1 g/day, in single, divided, or continuous doses (which dose may be adjusted for the patient's weight in kg, body surface area in m², and age in years).

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

### E. Metabolites of the Compounds

Also herein described are the *in vivo* metabolic products of the quinone prodrug compositions described herein, particularly β-lapachone prodrug compositions described herein. Metabolites of the prodrug compounds described herein include the active moiety released from the prodrug following administration to a subject, including the base quinone such as the β-lapachone compounds described herein. Such products may result for example from the oxidation, reduction, hydrolysis, amidation, esterification and the like of the administered compound, primarily due to enzymatic processes. Accordingly, there is herein described compounds produced by a process comprising contacting a quinone prodrug composition described herein with a mammalian tissue or a mammal for a period of time sufficient to yield a metabolic product thereof.

Such products typically are identified by preparing a radio-labeled (*e.g*. ¹⁴C and/or ³H) prodrug as herein described, administering it in a detectable dose (*e.g*., greater than about 0.5 mg/kg) to a mammal such as rat, mouse, guinea pig, monkey, or to man, allowing sufficient time for metabolism to occur (typically about 30 seconds to 30 hours), and isolating its conversion products from urine, blood, tumor, or other biological samples. These products are easily isolated since they are radio-labeled. The metabolite structures are determined in conventional fashion, *e.g*., by MS, MS/MS, or NMR analysis. In general, analysis of metabolites may be done in the same way as conventional drug metabolism studies well-known to those skilled in the art. The conversion products, so long as they are not otherwise found *in vivo,* are useful in diagnostic assays for therapeutic dosing of the compounds herein described even if they possess no biological activity of their own.

### F. Pharmaceutical Compositions

There is also herein described pharmaceutical compositions useful in the methods described herein. The pharmaceutical compositions may be formulated with pharmaceutically acceptable excipients such as carriers, solvents, stabilizers, adjuvants, diluents, etc., depending upon the particular mode of administration and dosage form. The pharmaceutical compositions should generally be formulated to achieve a physiologically compatible pH, and may range from a pH of about 3.0 to a pH of about 11.0, depending on the formulation, route of administration, and any other factors required to deliver a therapeutically effective dosage.

More particularly, the pharmaceutical compositions herein described comprise a therapeutically effective amount of at least one quinone prodrug composition herein described, together with one or more pharmaceutically acceptable excipients. When the pharmaceutical composition is formulated, the composition preferably comprises from about 0.1 mg/ml to about 50 mg/ml of the quinone prodrug composition.

Optionally, the pharmaceutical compositions may comprise a combination of quinone prodrug compositions herein described, or may include a second therapeutic agent useful in the treatment of cancer. Therapeutic amounts of second agents are generally known in the art or may be determined by the skilled clinician.

Formulations, *e.g*., for parenteral or oral administration, are most typically solids, liquid solutions, emulsions or suspensions, while inhaleable formulations for pulmonary administration are generally liquids or powders, with powder formulations being generally preferred. A preferred pharmaceutical composition may also be formulated as a lyophilized solid that is reconstituted with a physiologically compatible solvent prior to administration. Alternative pharmaceutical compositions may be formulated as syrups, creams, ointments, tablets, and the like.

The term "pharmaceutically acceptable excipient" refers to an excipient for administration of a pharmaceutical agent, such as the compounds herein described. The term refers to any pharmaceutical excipient that may be administered without undue toxicity. Pharmaceutically acceptable excipients are determined in part by the particular composition being administered, as well as by the particular method used to administer the composition. Accordingly, there exist a wide variety of suitable formulations of pharmaceutical compositions (*see, e.g.,* Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants such as ascorbic acid; chelating agents such as EDTA; carbohydrates such as dextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients.

The pharmaceutical compositions may be formulated in any form suitable for the intended method of administration. When intended for oral use for example, tablets, troches, lozenges, aqueous or oil suspensions, non-aqueous solutions, dispersible powders or granules (including micronized particles or nanoparticles), emulsions, hard or soft capsules, syrups or elixirs may be prepared. Compositions intended for oral use may be prepared according to any method known to the art for the manufacture of pharmaceutical compositions, and such compositions may contain one or more agents including sweetening agents, flavoring agents, coloring agents and preserving agents, in order to provide a palatable preparation.

Pharmaceutically acceptable excipients particularly suitable for use in conjunction with tablets include, for example, inert diluents, such as celluloses, calcium or sodium carbonate, lactose, calcium or sodium phosphate; disintegrating agents, such as croscarmellose sodium, cross-linked povidone, maize starch, or alginic acid; binding agents, such as povidone, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example celluloses, lactose, calcium phosphate or kaolin, or as soft gelatin capsules wherein the active ingredient is mixed with non-aqueous or oil medium, such as glycerin, propylene glycol, polyethylene glycol, peanut oil, liquid paraffin or olive oil.

Pharmaceutical compositions herein described may be formulated as suspensions comprising a compound herein described in admixture with at least one pharmaceutically acceptable excipient suitable for the manufacture of a suspension. Pharmaceutical compositions herein described may be formulated as dispersible powders and granules suitable for preparation of a suspension by the addition of suitable excipients.

Excipients suitable for use in connection with suspensions include suspending agents, such as sodium carboxymethylcellulose, methylcellulose, hydroxypropyl methylcelluose, sodium alginate, polyvinylpyrrolidone, gum tragacanth, gum acacia, dispersing or wetting agents such as a naturally occurring phosphatide (*e.g*., lecithin), a condensation product of an alkylene oxide with a fatty acid (*e.g*., polyoxyethylene stearate), a condensation product of ethylene oxide with a long chain aliphatic alcohol (*e.g*., heptadecaethyleneoxycethanol), a condensation product of ethylene oxide with a partial ester derived from a fatty acid and a hexitol anhydride (*e.g*., polyoxyethylene sorbitan monooleate); and thickening agents, such as carbomer, beeswax, hard paraffin or cetyl alcohol. The suspensions may also contain one or more preservatives such as acetic acid, methyl and/or n-propyl p-hydroxy-benzoate; one or more coloring agents; one or more flavoring agents; and one or more sweetening agents such as sucrose or saccharin.

The pharmaceutical compositions herein described may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth; naturally occurring phosphatides, such as soybean lecithin, esters or partial esters derived from fatty acids; hexitol anhydrides, such as sorbitan monooleate; and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

Additionally, the pharmaceutical compositions may be in the form of a sterile injectable preparation, such as a sterile injectable aqueous emulsion or oleaginous suspension. This emulsion or suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,2-propane-diol. The sterile injectable preparation may also be prepared as a lyophilized powder. Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or diglycerides. In addition, fatty acids such as oleic acid may likewise be used in the preparation of injectables.

### G. Combination Therapy

It is also possible to combine any quinone prodrug composition herein described with one or more other active agents useful in the treatment of the target disease or disorder such as cancer, including compounds, in a unitary dosage form, or in separate dosage forms intended for simultaneous or sequential administration to a patient in need of treatment. When administered sequentially, the combination may be administered in two or more administrations. It is possible to administer one or more compounds herein described and one or more additional active ingredients by different routes.

The skilled artisan will recognize that a variety of active ingredients may be administered in combination with the compounds herein described that may act to augment or synergistically enhance the activity of the quinone prodrug composition. Examples of second anticancer agents include, but are not limited to, taxane derivatives such as paclitaxel and docetaxol; gemcitabine (Gemzar®), other nucleoside and nucleotide anticancer agents; cisplatin (Platinol®); targeted agents such as imatnibmeasylate (Gleevec®) and trastuzumab (Herceptin®); or any other anticancer agent approved for therapeutic use in humans. Further anticancer agents useful in combination therapies are disclosed, *e.g*., in U.S. 2004/0087610 A1.

According to the methods herein described, the combination of active ingredients may be: (1) co-formulated and administered or delivered simultaneously in a combined formulation; (2) delivered by alternation or in parallel as separate formulations; or (3) by any other combination therapy regimen known in the art. When delivered in alternation therapy, the methods may comprise administering or delivering the active ingredients sequentially, *e.g*., in separate solution, emulsion, suspension, tablets, pills or capsules, or by different injections in separate syringes. In general, during alternation therapy, an effective dosage of each active ingredient is administered sequentially, *i.e*., serially, whereas in simultaneous therapy, effective dosages of two or more active ingredients are administered together. Various sequences of intermittent combination therapy may also be used.

To assist in understanding the present invention, the following Examples are included.

### EXAMPLES

The present invention is described in more detail with reference to the following examples . The examples illustrate the preparation of certain compounds of the invention, and the testing of these compounds *in vitro* and/or *in vivo.*

### A. Comparative Example 1: Synthesis of 5-(acetyloxy)-2,2-dimethyl-3,4-dihydro-2H-benzo[h]chromen-6-yl N-(tert-butoxycarbonyl)glycinate (1)

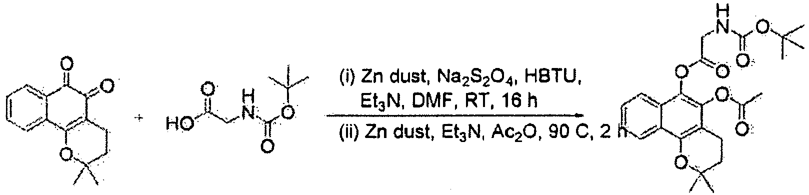

**5-(acetytoxy)-2,2-dimethyl-3,4-dihydro-2*H*-benzo[*h*]chromen-6-yl *N*-(*tert-*butoxycarbonyl)glycinate (1)**. A mixture of zinc dust (6.0 g, 91.7 mmol), 2,2-dimethyl-3,4-dihydro-2*H*-benzo[*h*]chromene-5,6-dione (6.0 g, 24.8 mmol), Na₂S₂O₄ (17.26 g, 99.1 mmol), *N-*(*tert*-butoxycarbonyl)glycine (8.77 g, 49.6 mmol), triethylamine (3.1 mL, 22.3 mmol), HBTU (18.79 g, 49.6 mmol) and DMF (100 mL) is stirred for 16 hours at room temperature. To the reaction mixture is then added EtOAc (300 mL). The reaction is filtered and the filtrate washed with H₂O (4 x 200 mL). The organic extract is dried with Na₂SO₄ and concentrated under reduced pressure. The residue is dissolved in acetic anhydride (30 mL) followed by the addition of zinc dust (3.0 g, 45.9 mmol) and thriethylamine (3.35 mL, 24.0 mmol). The reaction is heated at 90 °C with vigorous stirring and held for 2 hours. The reaction is allowed to cool and the solvent removed under reduced pressure. The residue is dissolved in EtOAc (200 mL) and washed with water (2 x 100 mL). The organic extract is dried with Na₂SO₄ and concentrated under reduced pressure. The crude product is purified by flash column chromatography (SiO₂, 2% EtOAc in dichloromethane) to afford about 60% pure final product. Crystallization of the partly pure solid from EtOAc/Hexane gives the desired product as pure white solid (3.2 g, 31%). M.p. = 177 °C; 400 MHz ¹H NMR (CDCl₃) δ: 8.21 (d, *J* = 8.4 Hz, 1H), 7.67 (d, *J* = 8.4 Hz, 1H), 7.46 (m, 2H), 5.13 (br, s, 1H), 4.30 (d, *J* =5.6 Hz, 2H), 2.68 (t, *J* = 6.6 Hz, 2H), 2.38 (s, 3H), 1.87 (t, *J* = 6.6 Hz, 2H), 1.48 (s, 9H), 1.42 (s, 6H); LCMS: 444 [M+H]; Calc. for C₂₄H₂₉NO₇: C 64.94, H 6.59, N 3.16; Found C 64.98, H 6.51, N 3.15.

### B. Examples 1 : Synthesis of 5-(acetyloxy)-2,2-dimethyl-3,4-dihydro-2H-benzo[h]chromen-6-yl glycinate hydrochloride (2)

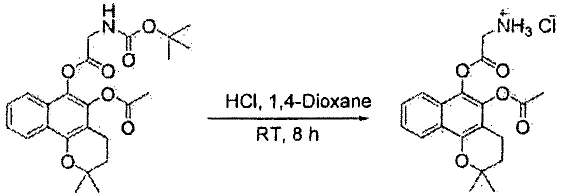

**Synthesis of 5-(acetytoxy)-2,2-dimethyl-3,4-dihydro-2*H*-benzo[*h*]chromen-6-yl glycinate hydrochloride (2 - Prodrug 1)**: To a solution of 5-(acetyloxy)-2,2-dimethyl-3,4-dihydro-2*H*-benzo[*h*]chromen-6-yl *N-*(*tert*-butoxycarbonyl)glycinate (**1**) (2.35 g, 4.0 mmol) in 1,4-dioxane (25 mL) is added a solution of hydrogen chloride gas in anhydrous 1,4-dioxane (4.0 M, 60 mL). The reaction is stirred at room temperature for 6 hours. The reaction is dried under reduced pressure. The product is obtained as a white solid (1.489 g, 95%) M.p. = 176-178 °C; 400 MHz ¹H NMR (DMSO-d₆) δ: 8.58 (br. s, 3H), 8.14 (m, 1H), 7.9 (m, 1H), 7.55 (m, 2H), 4.41 (s, 2H), 2.62 (t, *J*= 6.6 Hz, 2H), 2.41 (s, 3H), 1.88 (t, *J* = 6.6 Hz, 2H), 1.39 (s, 6H); LCMS: 344 [M+H]; Calc. for C₁₉H₂₁NO₅. 1.25 HCl: C 58.62, H 5.77, N 3.6; Found C 58.7, H 5.72, N 3.47.

### C. Comparative Example 2: Synthesis of 5-(acetyloxy)-2,2-dimethyl-3,4-dihydro-2H-benzo[h]chromen-6-yl N-(tert-butoxycarbonyl)-L-alaninate (3) and 6-(acetyloxy)-2,2-dimethyl-3,4-dihydro-2H-benzo[h]chromen-5-yl N-(tert-butoxycarbonyl)-L-alaninate (4)

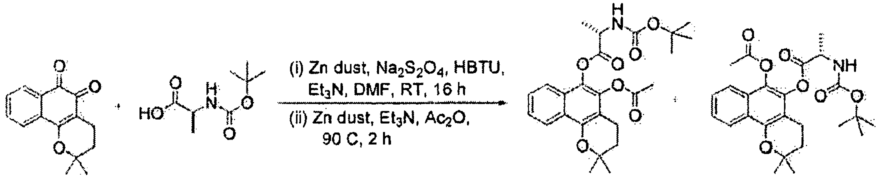

**5-(acetyloxy)-2,2-dimethyl-3,4-dihydro-2*H*-benzo[*h*]chromen-6-yl *N*-(*tert-*butoxycarbonyl)-L-alaninate (3) and 6-(acetyloxy)-2,2-dimethyl-3,4-dihydro-2*H-*benzo[*h*]chromen-5-yl *N*-(*tert*-butoxycarbonyl)-L-alaninate (4)**. The compounds 5-(acetyloxy)-2,2-dimethyl-3,4-dihydro-2*H*-benzo[*h*]chromen-6-yl *N*-(*tert*-butoxycarbonyl) -L-alaninate (**3**) and 6-(acetyloxy)-2,2-dimethyl-3,4-dihydro-2*H*-benzo[*h*]chromen-5-yl *N*-(*tert*-butoxycarbonyl)-L-alaninate (**4**) are synthesized as described in example 1 using zinc dust (2.0 g, 30.5 mmol), 2,2-dimethyl-3,4-dihydro-2*H*-benzo[*h*]chromene-5,6-dione (2.0 g, 8.26 mmol), Na₂S₂O₄ (5.75 g, 33.0 mmol), *N*-(*tert*-butoxycarbonyl)-L-alanine (3.12 g, 16.51 mmol), triethylamine (1.04 mL, 7.43 mmol), HBTU (6.26 g, 16.5 mmol) and DMF (20 mL) for the 1^{st} step. The acetylation step is carried out using zinc dust (1.0 g, 15.3 mmol), thriethylamine (1.04 mL, 7.43 mmol) and acetic anhydride (30 mL). Both the compounds are generated in the reaction. The crude mixture is purified by three consecutive flash column chromatography on SiO₂ (twice using a gradient from 10% EtOAc in hexanes to 25% EtOAc in hexanes once using 100% dichloromethane) to afford pure desired product (1.37 g, 36%) as mixture of isomers (**3:4**) in a ratio of 2.8:1, as established by ¹H NMR. M.p. = 94-98 °C; 400 MHz ¹H NMR (CDCl₃), major isomer δ: 8.21 (d, *J* = 8.8 Hz, 1H), 7.69 (d, *J* = 8.8 Hz, 1H), 7.45 (m, 2H), 5.10 (d, *J* = 8.4 Hz, 1H), 4.71 (dd, *J* = 7.6, 7.6 Hz, 1H), 2.68 (t, *J* = 6.8 Hz, 2H), 2.37 (s, 3H), 1.87 (t, *J* = 6.8 Hz, 2H), 1.67 (d, *J* = 7.2 Hz, 3H), 1.48 (s, 9H), 1.42 (s, 6H); minor isomer δ: 8.20 (d, *J*= 7.6 Hz, 1H), 7.67 (d, *J*= 7.6 Hz, 1H), 7.45 (m, 2H), 5.09 (d, *J* = 9.2 Hz, 1H), 4.64 (dd, *J* = 8.4, 7.6 Hz, 1H), 2.67 (t, *J*= 6.8 Hz, 2H), 2.43 (s, 3H), 1.87 (t, *J*= 6.8 Hz, 2H), 1.61 (d, *J* = 6.8 Hz, 3H), 1.48 (s, 9H), 1.42 (s, 6H); LCMS: 458 [M+H]; Calc. for C₂₅H₃₁NO₇ 0.7 H₂O 0.4 CH₂Cl₂: C 60.46, H 6.64, N 2.79; Found C 60.46, H 6.60, N 3.02.

### D. Example 2: Synthesis of 5-(acetyloxy)-2,2-dimethyl-3,4-dihydro-2H-benzo[h]chromen-6-yl L-alaninate hydrochloride (5) and 6-(acetyloxy)-2,2-dimethyl-3,4-dihydro-2H-benzo[h]chromen-5-yl L-alaninate hydrochloride (6)

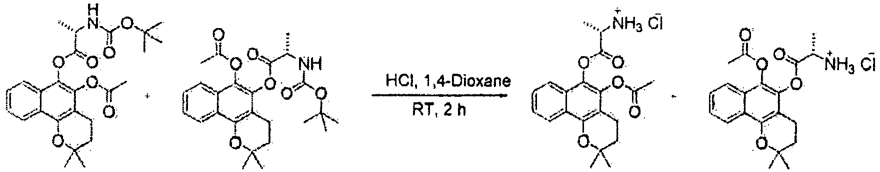

**5-(acetyloxy)-2,2-dimethyl-3,4-dihydro-2*H*-benzo[*h*]chromen-6-yl L-alaninate hydrochloride (5 - Prodrug 4) and 6-(acetyloxy)-2,2-dimethyl-3,4-dihydro-2*H*-benzo[*h*]chromen-5-yl L-alaninate hydrochloride (6)**. To a solution of 5-(acetyloxy)-2,2-dimethyl-3,4-dihydro-2*H*-benzo[*h*]chromen-6-yl *N*-(*tert-*butoxycarbonyl)-L-alaninate (**3**) and 6-(acetyloxy)-2,2-dimethyl-3,4-dihydro-2*H-*benzo[*h*]chromen-5-yl *N*-(*tert*-butoxycarbonyl)-L-alaninate (**4**) (1.3 g, 2.84 mmol) in 1,4-dioxane (5 mL) is added a solution of hydrogen chloride gas in anhydrous 1,4-dioxane (4.0 M, 20 mL). The reaction is stirred at room temperature for 30 minutes. The reaction is dried under reduced pressure resulting in a pale yellow solid. The solid is triturated with Et₂O to afford the desired product as an off white solid (1.11 g, 99%) and is a mixture of both the isomers 5-(acetyloxy)-2,2-dimethyl-3,4-dihydro-2*H-*benzo[*h*]chromen-6-yl L-alaninate hydrochloride (5) and 6-(acetyloxy)-2,2-dimethyl-3,4-dihydro-2*H*-benzo[*h*]chromen-5-yl L-alaninate hydrochloride (**6**). The ratio of the isomers **5:6** is determined to be 4:1 by ¹H NMR. M.p. = 229-230 °C; 400 MHz ¹H NMR (DMSO-d₆), major isomer δ: 8.80 (br. s, 3H), 8.15 (d, *J* = 8.4 Hz, 1H), 7.88 (d, *J* = 8.4 Hz, 1H), 7.57 (m, 2H), 4.69 (q, *J* = 7.2 Hz, 1H), 2.64 (m, 2H), 2.39 (s, 3H), 1.88 (t, *J* = 6.6 Hz, 2H), 1.69 (d, *J* = 7.2 Hz, 3H), 1.40 (s, 6H); minor isomer δ: 8.80 (br. s, 3H), 8.14 (d, *J* = 8.1 Hz, 1H), 7.79 (d, *J* = 8.0 MHz, 1H), 7.57 (m, 2H), 4.60 (q, *J*= 7.2 Hz, 1H), 2.64 (m, 2H), 2.45 (s, 3H), 1.88 (t, *J* = 6.6 Hz, 2H), 1.66 (d, *J* = 7.2 Hz, 3H), 1.39 (s, 6H); LCMS: 358 [M+H]; Calc. for C₂₀H₂₃NO₅ 1.11 HCl: C 60.32, H 6.11, N 3.52; Found C 60.40, H 6.17, N 3.43.

### E. Comparative Example 3: Synthesis of 5-(acetyloxy)-2,2-dimethyl-3,4-dihydro-2H-benzo[h]chromen-6-yl N-(tert-butoxycarbonyl)-L-valinate (7) and 6-(acetyloxy)-2,2-dimethyl-3,4-dihydro-2H-benzo[h]chromen-5-yl N-(tert-butoxycarbonyl)-L-valinate (8)

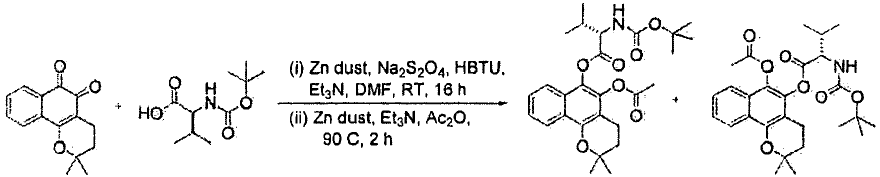

**5-(acetyloxy)-2,2-dimethyl-3,4-dihydro-2*H*-benzo[h]chromen-6-yl *N*-(*tert-*butoxycarbonyl)-L-valinate (7) and 6-(acetyloxy)-2,2-dimethyl-3,4-dihydro-2*H-*benzo[*h*]chromen-5-yl *N*-(*tert*-butoxycarbonyl)-L-valinate (8).** The compounds 5-(acetyloxy)-2,2-dimethyl-3,4-dihydro-2H-benzo[*h*]chromen-6-yl *N*-(*tert*-butoxycarbonyl) -L-valinate (**7**) and 6-(acetyloxy)-2,2-dimethyl-3,4-dihydro-2*H*-benzo[*h*]chromen-5-yl *N-*(*tert*-butoxycarbonyl)-L-valinate (**8**) are synthesized as described in example 1 using zinc dust (3.0 g, 46 mmol), 2,2-dimethyl-3,4-dihydro-2*H*-benzo[h]chromene-5,6-dione (3.0 g, 12.4 mmol), Na₂S₂O₄ (g, mmol), *N*-(*tert*-butoxycarbonyl)-L-valine (8.6 g, 49.5 mmol), triethylamine (1.5 mL, 10.7 mmol), HBTU (9.3 g, 24.6 mmol) and DMF (30 mL) for the 1^{st} step. The acetylation step is carried out using zinc dust (1.5 g, 23 mmol), thriethylamine (3.3 mL, 23.7 mmol) and acetic anhydride (30 mL). Both the compounds are generated in the reaction. The crude mixture is purified by flash column chromatography (SiO₂, 1% EtOAc in dichloromethane to 5% EtOAc in dichloromethane) to afford pure desired products (1.02 g, 17%) as mixture of isomers (**7:8**) in a ratio of 2.8:1 as established by ¹H NMR. Mixture of two isomers, ratio by NMR = 2.8:1. M.p. = 74-76 °C; 400 MHz ¹H NMR (CDCl₃), major isomer δ: 8.21 (d, *J*= 8.1 Hz, 1H), 7.70 (d, *J* = 7.7 Hz, 1H), 7.45 (m, 2H), 5.08 (d, *J* = 9.9 Hz, 1H), 4.65 (dd, *J =* 9.5, 4.4 Hz, 1H), 2.67 (t, *J* = 6.8 Hz, 2H), 2.54-2.40 (m, 1H), 2.35 (s, 3H), 1.87 (t, *J* = 6.8 Hz, 2H), 1.48 (s, 9H), 1.43 (s, 6H), 1.17 (d, *J* = 7.0 Hz, 3H), 1.09 (d, *J* = 7.0 Hz, 3H); minor isomer δ:8.21 (d, *J* = 8.1 Hz, 1H), 7.65 (d, *J* = 7.3 Hz, 1H), 7.45 (m, 2H), 5.07 (d, *J* = 11.7 Hz, 1H), 4.56 (dd, *J* = 9.9, 4.4 Hz, 1H), 2.67 (t, *J* = 6.8 Hz, 2H), 2.54-2.40 (m, 1H), 2.42 (s, 3H), 1.87 (t, *J* = 6.8 Hz, 2H), 1.48 (s, 9H), 1.41 (s, 6H), 1.14 (d, *J* = 7.0 Hz, 3H), 1.06 (d, *J* = 7.0 Hz, 3H).

### F. Example 3: Synthesis of 5-(acetyloxy)-2,2-dimethyl-3,4-dihydro-2H-benzo[h]chromen-6-yl L-valinate hydrochloride (9) and 6-(acetyloxy)-2,2-dimethyl-3,4-dihydro-2H-benzo[h]chromen-5-yl L-valinate hydrochloride (10)

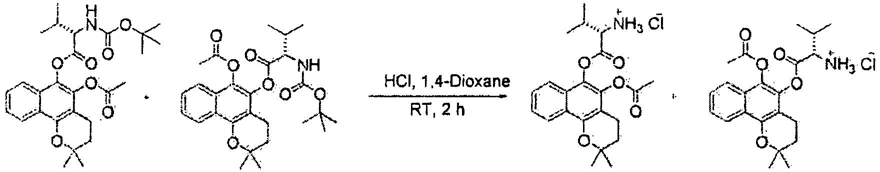

**5-(acetyloxy)-2,2-dimethyl-3,4-dihydro-2*H*-benzo[*h*]chromen-6-yl L-valinate hydrochloride (9 - Prodrug 2) and 6-(acetyloxy)-2,2-dimethyl-3,4-dihydro-2*H-*benzo[*h*]chromen-5-yl L-valinate hydrochloride (10).** To a solution of 5-(acetyloxy)-2,2-dimethyl-3,4-dihydro-2*H*-benzo[*h*]chromen-6-yl *N*-(*tert*-butoxycarbonyl)-L-valinate (**7**) and 6-(acetyloxy)-2,2-dimethyl-3,4-dihydro-2*H*-benzo[*h*]chromen-5-yl *N*-(*tert-*butoxycarbonyl)-L-valinate (**8**) (0.95 g, 1.95 mmol) in 1,4-dioxane (5 mL) is added a solution of hydrogen chloride gas in anhydrous 1,4-dioxane (4.0 M, 10 mL). The reaction is stirred at room temperature for 4 hours. The reaction is dried under reduced pressure. The product is obtained as a white solid (0.72 g, 95%) and is a mixture of both the isomers 5-(acetyloxy)-2,2-dimethyl-3,4-dihydro-2*H*-benzo[*h*]chromen-6-yl L-valinate hydrochloride (**9**) and 6-(acetyloxy)-2,2-dimethyl-3,4-dihydro-2*H*-benzo[*h*]chromen-5-yl L-valinate hydrochloride (**10**). The ratio of the isomers **9:10** is determined to be 4:1 by ¹H NMR. Mixture of two isomers, ratio by NMR = 4:1. M.p. = 149-151 °C; 400 MHz ¹H NMR (DMSO-d₆), major isomer δ: 8.79 (br. s, 3H), 8.15 (d, *J* = 6.8 Hz, 1H), 7.91 (d, *J* = 7.6 Hz, 1H), 7.60 (m, 2H), 4.59 (d, *J* = 3.2 Hz, 1H), 3.4-3.2 (m, 2H), 2.7-2.5 (m, 1H), 2.38 (s, 3H), 1.89 (m, 2H), 1.39 (s, 6H), 1.14 (m, 6H); minor isomer δ:8.79 (br. s, 3H), 8.15 (d, *J* = 6.8 Hz, 1H), 7.78 (d, *J* = 7.8 Hz, 1H), 7.60 (m, 2H), 4.48 (d, *J* = 3.6 Hz, 1H), 3.4-3.2 (m, 2H), 2.7-2.5 (m, 1H), 2.45 (s, 3H), 1.89 (m, 2H), 1.41 (s, 6H), 1.14 (m, 6H); LCMS: 386 [M+H]; Calc. for C₂₂H₂₇NO₅ 1.25 HCl: C 61.25, H 6.60, N 3.24; Found C 61.06, H 6.41, N 3.22.

### G. Example 4: Synthesis of 2-[5-(acetyloxy)-2,2-dimethyl-3,4-dihydro-2H-benzo[h]chromen-6-yl] -1-tert-butyl (2S)-pyrrolidine-1,2-dicarboxylate (11) and 2-[6-(acetyloxy)-2,2-dimethyl-3,4-dihydro-2H-benzo[h]chromen-5-yl] 1-tert-butyl (2S)-pyrrolidine-1,2-dicarboxylate (12)

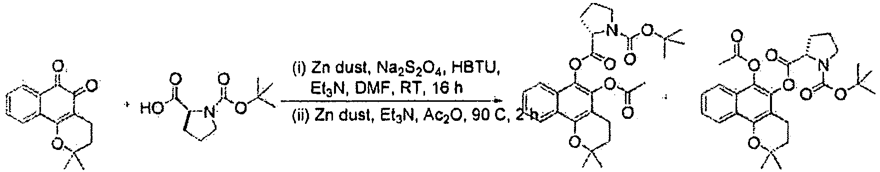

**2-[5-(acetyloxy)-2,2-dimethyl-3,4-dihydro-2*H*-benzo[*h*]chromen-6-yl] *1-tert-*butyl (2*S*)-pyrrolidine-1,2-dicarboxylate (11) and 2-[6-(acetyloxy)-2,2-dimethyl-3,4-dihydro-2*H*-benzo[*h*]chromen-5-yl] 1-*tert*-butyl (2*S*)-pyrrolidine-1,2-dicarboxylate (12)**. The compounds 2-[5-(acetyloxy)-2,2-dimethyl-3,4-dihydro-2*H*-benzo[h]chromen-6-yl] 1-*tert*-butyl (2*S*)-pyrrolidine-1,2-dicarboxylate (**11**) and 2-[6-(acetyloxy)-2,2-dimethyl-3,4-dihydro-2*H*-benzo[*h*]chromen-5-yl] 1-*tert*-butyl (2*S*)-pyrrolidine-1,2-dicarboxylate (**12**) are synthesized as described in example 1 using zinc dust (3.0 g, 45.9 mmol), 2,2-dimethyl-3,4-dihydro-2*H*-benzo[h]chromene-5,6-dione (3.0 g, 12.39 mmol), Na₂S₂O₄ (8.77 g, 49.6 mmol), *N*-(*tert*-butoxycarbonyl)-L-proline (8.0 g, 37.2 mmol), triethylamine (2.6 mL, 18.6 mmol), HBTU (14.1 g, 37.2 mmol) and DMF (80 mL) for the 1^{st} step. The acetylation step is carried out using zinc dust (1.5 g, 18.6 mmol), thriethylamine (2.6 mL, 18.6 mmol) and acetic anhydride (40 mL). Both the compounds are generated in the reaction. The two isomers in the crude mixture are separated by two consecutive flash column chromatography on SiO₂ (1^{st} purification is carried out using a step gradient from 100% dichloromethane to 2% EtOAc in dichloromethane and 2^{nd} purification using a step gradient from 15% EtOAc in hexanes to 20% EtOAc in hexanes). The purification affords 2-[5-(acetyloxy)-2,2-dimethyl-3,4-dihydro-2*H-*benzo[*h*]chromen-6-yl] 1-*tert*-butyl (2*S*)-pyrrolidine-1,2-dicarboxylate (**11**) (1.22 g) and 2-[6-(acetyloxy)-2,2-dimethyl-3,4-dihydro-2*H*-benzo[*h*]chromen-6-yl] 1-*tert*-butyl (2*S*)-pyrrolidine-1,2-dicarboxylate (**12**) (0.21 g) as a pure white solids (combined yield: 24%). 2-[5-(acetyloxy)-2,2-dimethyl-3,4-dihydro-2*H*-benzo[*h*]chromen-6-yl] 1-*tert*-butyl (2*S*)-pyrrolidine-1,2-dicarboxylate (**11**): M.p. = 83°C; 400 MHz ¹H NMR (CDCl₃) δ: 8.18 (d, *J* = 8.4 Hz, 1H), 7.9-7.7 (m, 1H), 7.43 (m, 2H), 4.70 (d, *J* = 8.8 Hz, 1H), 3.65-3.56 (m, 2H), 3.5-3.45 (m, 1H), 2.71-2.60 (m, 2H), 2.5-2.2 (m, 1H), 2.41 (s, 3H), 2.05-2.00 (m, 2H), 2.0-1.85 (m, 2H), 1.50 (s, 9H), 1.41 (m, 6H); LCMS: 484 [M+H]; Calc. for C₂₇H₃₃NO₇: C 67.00, H 6.88, N 2.90; Found C 67.14, H 6.78, N 2.85. 2-[6-(acetyloxy)-2,2-dimethyl-3,4-dihydro-2*H*-benzo[*h*]chromen-6-yl] 1-*tert*-butyl (2*S*)-pyrrolidine-1,2-dicarboxylate (**12**): M.p. = 84°C; 400 MHz ¹H NMR (CDCl₃) δ: 8.19 (d, *J* = 6.8 Hz, 1H), 7.7-7.6 (m, 1H), 7.44 (m, 2H), 4.63 (m, 1H), 3.65-3.56 (m, 2H), 3.5-3.4 (m, 1H), 2.73-2.67 (m, 2H), 2.5-2.2 (m, 1H), 2.47 (s, 3H), 2.1-1.95 (m, 2H), 1.86 (t, *J* = 6.6 Hz, 2H), 1.48 (s, 9H), 1.43-1.40 (m, 6H); LCMS: 484 [M+H]; Calc. for C₂₇H₃₃NO₇: C 67.00, H 6.88, N 2.90; Found C 67.32, H 6.58, N 2.84.

### H. Example 5: Synthesis of 5-(acetyloxy)-2,2-dimethyl-3,4-dihydro-2H-benzo[h]chromen-6-yl L-prolinate hydrochloride (13)

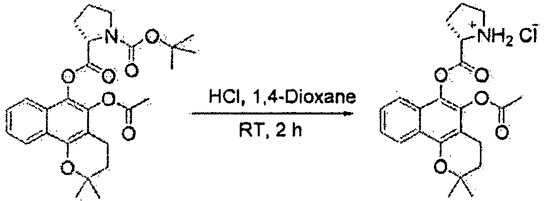

**5-(acetyloxy)-2,2-dimethyl-3,4-dihydro-2*H*-benzo[*h*]chromen-6-yl L-prolinate hydrochloride (13 - Prodrug 5).** To a solution of 2-[5-(acetyloxy)-2,2-dimethyl-3,4-dihydro-2*H*-benzo[*h*]chromen-6-yl] 1-*tert*-butyl (2*S*)-pyrrolidine-1,2-dicarboxylate (**11**) (1.2 g, 2.5 mmol) in 1,4-dioxane (5 mL) is added a solution of hydrogen chloride gas in anhydrous 1,4-dioxane (4.0 M, 10 mL). The reaction is stirred at room temperature for 4 hours. The reaction is dried under reduced pressure. The desired product is obtained as a white solid (1.015 g, 93%) M.p. = 125-130 °C; 400 MHz ¹H NMR (CDCl₃) δ: 8.81 (br. s, 2H), 8.18 (d, *J* = 8.8 Hz, 1H), 7.65 (d, *J* = 7.6 Hz, 1H), 7.44 (m, 2H), 4.81 (br. s, I H), 3.8-3.6 (m, 1H), 3.46 (br. s, 2H), 2.65-2.5 (m, 1H), 2.57 (t, *J* = 6.4 Hz, 2H), 2.4-2.3 (m, 1H), 2.38 (s, 3H), 2.15-2.00 (m, 2H), 1.85-1.70 (m, 1H), 1.77 (t, *J* = 6.4 Hz, 2H), 1.36 (s, 3H), 1.35 (s, 3H); LCMS: 384 [M+H]; Calc. for C₂₂H₂₅NO₅ 1.58 HCl: C 59.86, H 6.07, N 3.18; Found C 59.91, H 6.09, N 2.91.

### I. Comparative Example 4: Synthesis of 6-(acetyloxy)-2,2-dimethyl-3,4-dihydro-2H-benzo[h]chromen-5-yl L-prolinate hydrochloride (14)

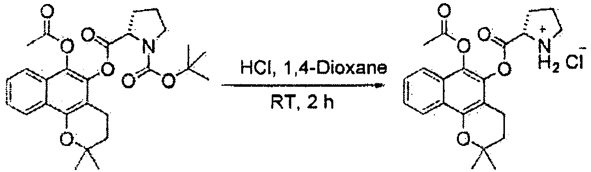

**6-(acetyloxy)-2,2-dimethyl-3,4-dihydro-2*H*-benzo[*h*]chromen-5-yl L-prolinate hydrochloride (14).** To a solution of 2-[6-(acetyloxy)-2,2-dimethyl-3,4-dihydro-2*H*-benzo[*h*]chromen-6-yl] 1-*tert*-butyl (2*S*)-pyrrolidine-1,2-dicarboxylate (**12**) (0.215 g, 0.45 mmol) in 1,4-dioxane (5.0 mL) is added a solution of hydrogen chloride gas in anhydrous 1,4-dioxane (4.0 M, 5 mL). The reaction is stirred at room temperature for 2 hours. The reaction is dried under reduced pressure. The desired product is obtained as a white solid (0.152 g, 77%) M.p. = 100-110 °C; 400 MHz ¹H NMR (CDCl₃) δ: 8.21 (d, *J* = 9.2 Hz, 1H), 7.63 (d, *J* = 7.2 Hz, 1H), 7.46 (m, 2H), 4.76 (br. s, I H), 3.85-3.4 (m, 3H), 2.66 (m, 2H), 2.42 (m, 3H), 2.17 (m, 2H), 1.84 (m, 2H), 1.48 (m, 2H), 1.39 (s, 6H); LCMS: 384 [M+H]; Calc. for C₂₂H₂₅NO₅ 1.78 HCl: C 58.88, H 6.02, N 3.12; Found C 58.96, H 6.17, N 2.84.

### J. Comparative Example 5: Synthesis of 4-[5-(acetyloxy)-2,2-dimethyl-3,4-dihydro-2H-benzo[h]chromen-6-yl] 1-tert-butyl piperidine-1,4-dicarboxylate (15) and 4-[6-acetyloxy)-2,2-dimethyl-3,4-dihydro-2H-benzo[h]chomen-5-yl] 1-tert-butyl piperidine-1,4-dicarboxylate (16)

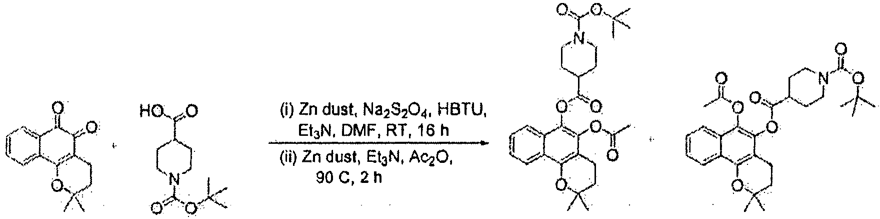

**4-[5-(acetyloxy)-2,2-dimethyl-3,4-dihydro-2*H*-benzo[*h*]chromen-6-yl] 1-*tert-*butyl piperidine-1,4-dicarboxylate (15) and 4-[6-(acetyloxy)-2,2-dimethyl-3,4-dihydro-2*H*-benzo[*h*]chromen-5-yl] 1-*tert*-butyl piperidine-1,4-dicarboxylate (16).** The compounds 4-[5-(acetyloxy)-2,2-dimethyl-3,4-dihydro-2*H*-benzo[*h*]chromen-6-yl] 1-*tert*-butyl piperidine-1,4-dicarboxylate (**15**) and 4-[6-(acetyloxy)-2,2-dimethyl-3,4-dihydro-2*H*-benzo[*h*]chromen-5-yl] 1-*tert*-butyl piperidine-1,4-dicarboxylate (**16**) are synthesized as described in example 1 using zinc dust (4.0 g, 61.2 mmol), 2,2-dimethyl-3,4-dihydro-2*H*-benzo[*h*]chromene-5,6-dione (4.0 g, 16.5 mmol), Na₂S₂O₄ (11.7 g, 66.1 mmol), 1-(*tert*-butoxycarbonyl)piperidine-4-carboxylic acid (9.46 g, 41.3 mmol), triethylamine (2.6 mL, 18.2 mmol), HBTU (15.7 g, 41.3 mmol) and DMF (100 mL) for the 1^{st} step. The acetylation step is carried out using zinc dust (2.0 g, 30.6 mmol), thriethylamine (2.6 mL, 18.2 mmol) and acetic anhydride (40 mL). Both the compounds are generated in the reaction. The crude mixture is purified by flash column chromatography (SiO₂, step gradient from 100% dichloromethane to 2% EtOAc in dichloromethane) to afford pure desired product (3.2 g, 39%) as mixture of isomers (**15:16**) in a ratio of 3:1 as established by ¹H NMR. Mixture of two isomers, ratio by NMR = 3:1. M.p. = 75-78 °C; 400 MHz ¹H NMR (CDCl₃), major isomer δ: 8.21 (d, *J*= 7.6 Hz, 1H), 7.61 (d, *J* = 7.6 Hz, 1H), 7.45 (m, 2H), 4.13 (m, 2H), 3.0-2.8 (m, 3H), 2.67 (t, *J*= 6.8 Hz, 2H), 2.34 (s, 3H), 2.2-2.0 (m, 2H), 1.95-1.8 (m, 4H), 1.48 (s, 9H), 1.42 (s, 6H); minor isomer δ: 8.21 (d, *J* = 7.6 Hz, 1H), 7.61 (d, *J* = 8.0 Hz, 1H), 7.45 (m, 2H), 4.13 (m, 2H), 3.0-2.8 (m, 3H), 2.64 (t, *J* = 7.6 Hz, 2H), 2.41 (s, 3H), 2.2-2.0 (m, 2H), 1.95-1.8 (m, 4H), 1.48 (s, 9H), 1.42 (s, 6H); LCMS: 498 [M+H]; Calc. for C₂₈H₃₅NO₇: C 67.53, H 7.09, N 2.81; Found C 67.30, H 6.81, N 2.76.

### K. Comparative Example 6: Synthesis of 5-(acetyloxy)-2,2-dimethyl-3,4-dihydro-2H-benzo[h]chromen-6-yl piperidíne-4-carboxylate hydrochloride (17) and 6-(acetyloxy)-2,2-dimethyl-3,4-dihydro-2H-benzo[h]chromen-5-yl piperidine-4-carboxylate hydrochloride (18)

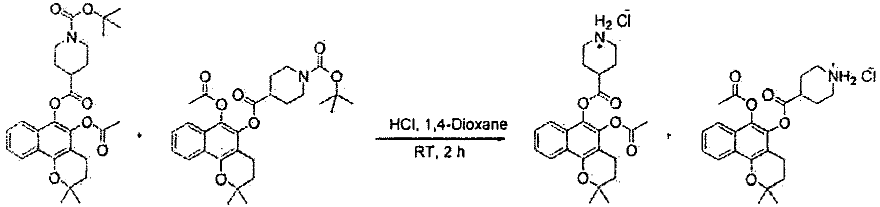

**5-(acetyloxy)-2,2-dimethyl-3,4-dihydro-2*H*-benzo[*h*]chromen-6-yl piperidine-4-carboxylate hydrochloride (17) and 6-(acetyloxy)-2,2-dimethyl-3,4-dihydro-2*H-*benzo[*h*]chromen-5-yl piperidine-4-carboxylate hydrochloride (18)**. To a solution of 4-[5-(acetyloxy)-2,2-dimethyl-3,4-dihydro-2*H*-benzo[h]chromen-6-yl] 1-*tert*-butyl piperidine-1,4-dicarboxylate (**15**) and 4-[6-(acetyloxy)-2,2-dimethyl-3,4-dihydro-2*H-*benzo[*h*]chromen-5-yl] 1-*tert*-butyl piperidine-1,4-dicarboxylate (**16**) (1.586 g, 3.18 mmol) in 1,4-dioxane (10 mL) is added a solution of hydrogen chloride gas in anhydrous 1,4-dioxane (4.0 M, 20 mL). The reaction is stirred at room temperature for 2 hours. The reaction is dried under reduced pressure. The product is obtained as a white solid (1.31 g, 94%) and is a mixture of both the isomers 5-(acetyloxy)-2,2-dimethyl-3,4-dihydro-2*H-*benzo[h]chromen-6-yl piperidine-4-carboxylate hydrochloride (**17**) and 6-(acetyloxy)-2,2-dimethyl-3,4-dihydro-2*H*-benzo[*h*]chromen-5-yl piperidine-4-carboxylate hydrochloride (**18**). The ratio of the isomers **17:18** is determined to be 3:1 by ¹H NMR. Mixture of two isomers, ratio by NMR = 3:1. M.p. = 225-229 °C; 400 MHz ¹H NMR (DMSO-d₆), major isomer δ: 8.87 (br. s, 2H), 8.12 (d, J *=* 7.6 Hz, 1H), 7.69 (d, *J* = 8.0 Hz, 1H), 7.50 (m, 2H), 3.4-3.3 (m, 1H), 3.21 (tt, *J* = 11.4, 3.8 Hz, 2H), 3.02 (m, 2H), 2.65-2.55 (m, 2H), 2.37 (s, 3H), 2.3-2.15 (m, 2H), 2.0-1.9 (m, 2H), 1.87 (t, *J* = 6.6 Hz, 2H), 1.38 (s, 6H); minor isomer δ: 8.87 (br. s, 2H), 8.12 (d, *J* = 7.6 Hz, 1H), 7.77 (d, *J* = 7.2 Hz, 1H), 7.50 (m, 2H), 3.4-3.3 (m, 1H), 3.12 (tt, *J* = 11.3, 3.8 Hz, 2H), 3.02 (m, 2H), 2.65-2.55 (m, 2H), 2.43 (s, 3H), 2.3-2.15 (m, 2H), 2.0-1.9 (m, 2H), 1.87 (t, *J* = 6.6 Hz, 2H), 1.38 (s, 6H); LCMS: 398 [M+H]; Calc. for C₂₃H₂₇NO₅ 1.12 HCl: C 62.97, H 6.47, N 3.20; Found C 63.04, H 6.71, N 3.23.

### L. Comparative Example 7: Synthesis of 5-(acetyloxy)-2,2-dimethyl-3,4-dihydro-2H-benzo[h]chromen-6-yl isonicotinate hydrochloride (19) and 6-(acetyloxy)-2,2-dimethyl-3,4-dilydro-2H-benzo[h]chromen-5-yl) isonicotinate hydrochloride (20)

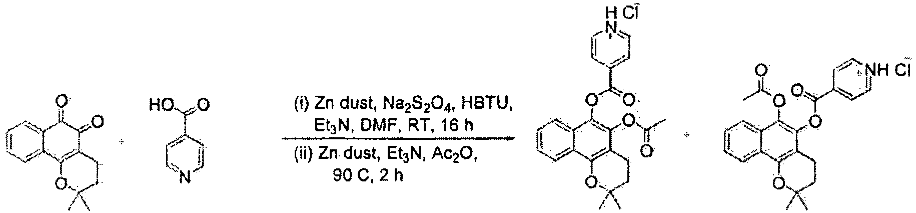

**5-(acetyloxy)-2,2-dimethyl-3,4-dihydro-2*H-*benzo[*h*]chromen-6-yl isonicotinate hydrochloride (19) and 6-(acetyloxy)-2,2-dimethyl-3,4-dihydro-2*H-*benzo[*h*]chromen-5-yl isonicotinate hydrochloride (20)**. The compounds 5-(acetyloxy)-2,2-dimethyl-3,4-dihydro-2*H-*benzo[*h*]chromen-6-yl isonicotinate hydrochloride (**19**) and 6-(acetyloxy)-2,2-dimethyl-3,4-dihydro-2*H-*benzo[*h*]chromen-5-yl isonicotinate hydrochloride (**20**) are synthesized as described in example 1 using zinc dust (1.5 g, 22.9 mmol), 2,2-dimethyl-3,4-dihydro-2*H-*benzo[*h*]chromene-5,6-dione (1.5 g, 6.2 mmol), Na₂S₂O₄ (4.32 g, 24.77 mmol), isonicotinic acid (1.52 g, 12.4 mmol)), triethylamine (0.78 mL, 5.57 mmol), HBTU (4.7 g, 12.38 mmol) and DMF (15 mL) for the 1^{st} step. The acetylation step is carried out using zinc dust (0.75 g, 11.5 mmol), thriethylamine (0.9 mL, 6.4 mmol) and acetic anhydride (10 mL). Both the compounds are generated in the reaction. The crude mixture is purified by flash column chromatography (SiO₂ using a gradient from 100% dichloromethane to 5% EtOAc in dichloromethane) to afford pure desired free-base of the product (1.038 g) as mixture of isomers. The free-base of the product is dissolved in Et₂O and treated with a solution of hydrogen chloride gas in anhydrous 1.,4-dioxane (4.0 M, 1.2 mL) for 15 minutes at room temperature. The desired hydrogen chloride salt of the product separates out as a pale yellow solid (0.973g, 36%), which is filtered and dried under reduced pressure. The desired product is a mixture of isomers (**19:20**) and the ratio is determined to be 4:1 by ¹H NMR. Mixture of two isomers, ratio by NMR = 4:1. M.p. = 228-232 °C; 400 MHz ¹H NMR (DMSO-d₆), major isomer δ: 9.05-8.95 (m, 2H), 8.2-8.15 (m, 2H), 8.12 (m, 1H), 7.85-7.75 (m, 1H), 7.62-7.50 (m, 2H), 2.67 (t, *J* = 6.8 Hz, 2H), 2.22 (s, 3H), 1.90 (t, *J* = 6.6 Hz, 2H), 1.41 (s, 6H); minor isomer δ: 9.05-8.95 (m, 2H), 8.2-8.15 (m, 2H), 8.12 (m, 1H), 7.85-7.75 (m, 1H), 7.62-7.50 (m, 2H), 2.67 (t, *J*= 6.8 Hz, 2H), 2.29 (s, 3H), 1.88 (t, *J* = 6.8 Hz, 2H), 1.41 (s, 6H); LCMS: 392 [M+H]; Calc. for C₂₃H₂₁NO₅. 1.21 HCl: C 63.37, H 5.14, N 3.22; Found C 63.41, H 5.36, N 3.16.

### M. Comparative Example 8: Synthesis of 5-(acetyloxy)-2.2-dimethy)-3,4-dihydro-2H-benzo[h]chromen-6-yl nicotinate hydrochloride (21) and 6-(acetyloxy)-2,2-dimethyl-3,4-dihydro-2H-benzo[h]chromen-5-yl nicotinate hydrochloride (22)

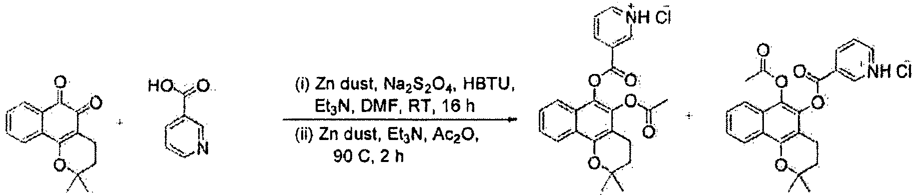

**5-(acetyloxy)-2,2-dimethyl-3,4-dihydro-2*H-*benzo[*h*]chromen-6-yl nicotinate hydrochloride (21-Prodrug 9) and 6-(acetytoxy)-2,2-dimethyl-3,4-dihydro-2*H-*benzo[*h*]chromen-5-yl nicotinate hydrochloride (22)**. The compounds 5-(acetyloxy)-2,2-dimethyl-3,4-dihydro-2*H* benzo[*h*]chromen-6-yl nicotinate hydrochloride (**21**) and 6-(acetyloxy)-2,2-dimethyl-3,4-dihydro-2*H*-benzo[*h*]chromen-5-yl nicotinate hydrochloride (**22**) are synthesized as described in example 1 using zinc dust (1.5 g, 22.9 mmol), 2,2-dimethyl-3,4-dihydro-2*H-*benzo[*h*]chromene-5,6-dione (1.5 g, 6.2 mmol), Na₂S₂O₄ (4.32 g, 24.77 mmol)), nicotinic acid (1.52 g, 12.4 mmol)), triethylamine (0.78 mL, 5.57 mmol), HBTU (4.7 g, 12.38 mmol) and DMF (15 mL) for the 1^{st} step. The acetylation step is carried out using zinc dust (0.75 g, 11.5 mmol), thriethylamine (0.9 mL, 6.4 mmol) and acetic anhydride (10 mL). Both the compounds are generated in the reaction. The crude mixture is purified by flash column chromatography (SiO₂, using a gradient from 100% dichloromethane to 5% EtOAc in dichloromethane) to afford pure desired free-base of the product (1.184 g) as mixture of isomers. The free-base of the product is dissolved in Et₂O and treated with a solution of hydrogen chloride gas in anhydrous 1,4-dioxane (4.0 M, 1.2 mL) for 15 minutes at room temperature. The desired hydrogen chloride salt of the product is separated out as a pale yellow solid (1.123 g, 41%), which is filtered and dried under reduced pressure. The desired product is a mixture of isomers (**21:22**) and the ratio is determined to be 3.3:1 by ¹H NMR. Mixture of two isomers, ratio by NMR = 3.3:1. M.p. = 214-218 °C; 400 MHz ¹H NMR (DMSO-d₆), major isomer δ: 9.84 (d, *J*= 2.0 Hz, 1H), 9.01-8.98 (m, 1H), 8.62-8.55 (m, 1H), 8.20-8.10 (m, 1H), 7.85-7.70 (m, 2H), 7.65-7.50 (m, 2H), 2.67 (t, *J*= 6.6 Hz, 2H), 2.22 (s, 3H), 1.90 (t, *J* = 6.4 Hz, 2H), 1.41 (s, 6H); minor isomer δ: 9.32 (d, *J =* 2.4 Hz, 1H), 9.01-8.98 (m, 1H), 8.62-8.55 (m, 1H), 8.20-8.10 (m, 1H), 7.85-7.70 (m, 2H), 7.65-7.50 (m, 2H), 2.71 (t, *J* = 6.4 Hz, 2H), 2.28 (s, 3H), 1.88 (t, *J*= 6.8 Hz, 2H), 1.41 (s, 6H); LCMS: 392 [M+H]; Calc. for C₂₃H₂₁NO₅ 1.4 HCl: C 62.38, H 5.10, N 3.17; Found C 62.42, H 5.01, N 3.15.

### N. Comparative Example 9: Synthesis of tert-butyl 4-(2-{[5-(acetyloxy)-2,2-dimethyl-3,4-dihydro-2H-benzo[h]chromen-6-yl]oxy}-2-oxoethyl)piperidine-1-carboxylate (23) and tert-t-butyl 4-(2-{[6-(acetyloxy)-2,2-dimethyl-3,4-dihydro-2H-benzo[h]chromen-5-yl]oxy}-2-oxoethyl)piperidine-1-carboxylate (24)

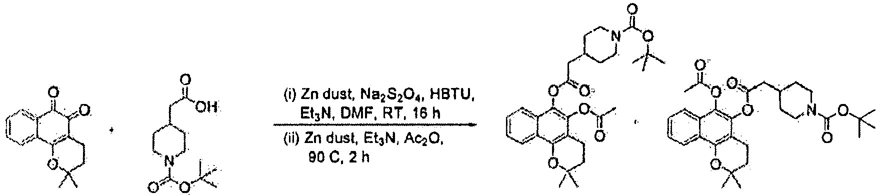

***tert-*butyl 4-(2-{[5-(acetyloxy)-2,2-dimethyl-3,4-dihydro-2*H-*benzo[*h*]chromen-6-yl]oxy}-2-oxoethyl)piperidine-1-carboxylate (23) and *tert-*butyl 4-(2-{[6-(acetyloxy)-2,2-dimethyl-3,4-dihydro-2*H* benzo[*h*]chromen-5-yl]oxy}-2-oxoethyl)piperidine-1-carboxylate (24)**. The compounds *tert-*butyl 4-(2-{[5-(acetyloxy)-2,2-dimethyl-3,4-dihydro-2*H*-benzo[*h*]chromen-6-yl]oxy}-2-oxoethyl)piperidine-1-carboxylate (**23**) and *tert-*butyl 4-(2-{[6-(acetyloxy)-2,2-dimethyl-3,4-dihydro-2*H*-benzo[*h*]chromen-5-yl]oxy}-2-oxoethyl)piperidine-1-carboxylate (**24**) are synthesized as described in scheme 1a using zinc dust (2.0 g, 30.5 mmol), 2,2-dimethyl-3,4-dihydro-2*H*-benzo[*h*]chromene-5,6-dione (2.0 g, 8.3 mmol), Na₂S₂O₄ (5.8 g, 33.0 mmol), [1-(*tert-*butoxycarbonyl)piperidin-4-yl]acetic acid (2.36 g, 1.6:5 mmol), triethylamine (1.04 mL, 7.4 mmol), HBTU (6.26 g, 16.5 mmol) and DMF (20 mL) for the 1^{st} step. The acetylation step is carried out using zinc dust (1.0 g, 15.3 mmol), thriethylamine (1.2 mL, 8.3 mmol) and acetic anhydride (15 mL). Both the compounds are generated in the reaction. The crude mixture is purified using two consecutive flash column chromatography (SiO₂ 1^{st} purification is carried out using linear gradient from 50% dichloromethane in hexanes to 100% dichloromethane. 2^{nd} purification using 2% EtOAc in dichloromethane) to afford pure desired product (1.42 g, 33%) as mixture of isomers (**23:24**) in a ratio of 2.7:1 as established by ¹H NMR. Mixture of two isomers, ratio by NMR = 2.7:1. M.p. = 70-72 °C; 400 MHz ¹H NMR (CDCl₃), major isomer δ: 8.21 (d, *J* = 8.0 Hz, 1H), 7.63 (d, *J* = 7.8 Hz, 1H), 7.50-7.40 (m, 2H), 4.10-4.00 (m, 2H), 2.78 (m, 2H), 2.70-2.60 (m, 4H), 2.34 (s, 3H), 2.20-2.05 (m, 1H), 1.87 (t, *J =* 6.8 Hz, 2H), 1.90-1.80 (m, 2H), 1.47 (s, 9H), 1.42 (s, 6H), 1.35-1.20 (m, 2H); minor isomer δ: 8.20 (d, *J =* 7.6 Hz, 1H), 7.67 (d, *J* = 7.6 Hz, 1H), 7.50-7.40 (m, 2H), 4.10-4.00 (m, 2H), 2.78 (m, 2H), 2.70-2.60 (m, 4H), 2.41 (s, 3H), 2.20-2.05 (m, 1H), 1.87 (t, *J* = 6.8 Hz, 2H), 1.90-1.80 (m, 2H), 1.47 (s, 9H), 1.42 (s, 6H), 1.35-1.20 (m, 2H); LCMS: 512 [M+H]; Calc. for C₂₉H₃₇NO₇: C 68.02, H 7.29, N 2.74; Found C 68.09, H 6.94, N 2.70.

### O. Comparative Example 10: Synthesis of 5-(acetyloxy)-2,2-dimethyl-3,4-dihydro-2H-benzo[h]chromen-6-ylpiperidin-4-ylacetate hydrochloride (25) and 6-(acetyloxy)-2,2-dimethyl-3,4-dihydro-2H-benzo[h]chromen-5-yl piperidin-4-ylacetate hydrochloride (26)

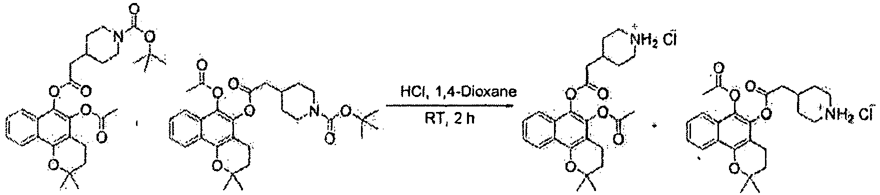

**5-(acetyloxy)-2,2-dimethyl-3,4-dihydio-2*H-*benzo[*h*]chromen-6-yl piperidin-4-ylacetate hydrochloride (25 - Prodrug 7) and 6-(acetyloxy)-2,2-dimethyl-3,4-dihydro-2*H*-benzo[*h*]chromen-5-yl piperidin-4-ylacetate hydrochloride (26)**. To a solution of *tert-*butyl 4-(2-{[5-(acetyloxy)-2,2-dimethyl-3,4-dihydro-2*H-*benzo[*h*]chromen-6-yl]oxy}-2-oxoethyl)piperidine-1-carboxylate (**23**) and *tert-*butyl *4-*(2-{[6-(acetyloxy)-2,2-dimethyl-3,4-dihydro-2*H*-benzo[*h*]chromen-5-yl]oxy}-2-oxoethyl)piperidine-1-carboxylate (**24**) (1.36 g, 2.66 mmol) in 1,4-dioxane (10 mL) is added a solution of hydrogen chloride gas in anhydrous 1,4-dioxane (4.0 M, 20 mL). The reaction is stirred at room temperature for 30 minutes. The reaction is dried under reduced pressure. The product is obtained as a white solid (1.09 g, 92%) and is a mixture of both the isomers 5-(acetyloxy)-2,2-dimethyl-3,4-dihydro-2*H-*benzo[*h*]chromen-6-yl piperidin-4-ylacetate hydrochloride (25) and 6-(acetyloxy)-2,2-dimethyl-3,4-dihydro-2*H* benzo[*h*]chromen-5-yl piperidin-4-ylacetate hydrochloride (26). The ratio of the isomers **25:26** is determined to be 2.6:1 by ¹H NMR. Mixture of two isomers, ratio by NMR = 2.6:1. M.p. = 207-210 °C; 400 MHz ¹H NMR (DMSO-d₆), major isomer δ: 9.02 (br. s, 2H), 8.10 (d, *J* = 7.2 Hz, 1H), 7.70 (d, *J* = 8.0 Hz, 1H), 7.60-7.45 (m, 2H), 3.27 (d, *J*= 12.8 Hz, 2H), 2.91 (t, *J =* 12.6 Hz, 2H), 2.79 (d, *J* = 6.8 Hz, 2H), 2.60 (t, *J* = 6.4 Hz, 2H), 2.35 (s, 3H), 2.20-2.05 (m, 1H), 1.95-1.80 (m, 4H), 1.60-1.45 (m, 2H), 1.37 (s, 6H); minor isomer δ: 9.02 (br. s, 2H), 8.10 (d, *J =* 7.2 Hz, 1H), 7.75 (d, *J* = 7.6 Hz, 1H), 7.60-7.45 (m, 2H), 3.27 (d, *J* = 1.2.8 Hz, 2H), 2.91 (t, *J* = 12.6 Hz, 2H), 2.69 (d, *J* = 6.8 Hz, 2H), 2.60 (t, *J* = 6.4 Hz, 2H), 2.41 (s, 3H), 2.20-2.05 (m, 1H), 1.95-1.80 (m, 4H), 1.60-1.45 (m, 2H), 1.37 (s, 6H); LCMS: 412 [M+H]; Calc. for C₂₄H₂₉NO₅ 1.3 HCl: C 62.76, H 6.65, N 3.05; Found C 62.58, H 6.78, N 3.02.

### P. Comparative Example 1.1: Synthesis of 5-(acetyloxy)-2,2-dimethyl-3A-dihydro-2H-benzo[h]chromen-6-yl N-(tert-butoxycarbonyl)-2-methylalaninate (27)

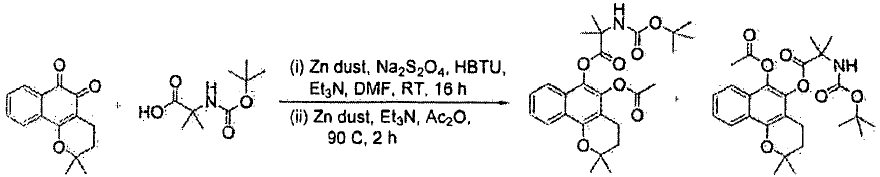

**5-(acetyloxy)-2,2-dimethyl-3,4-dihydro-2*H-*benzo[*h*]chromen-6-yl *N-*(*tert-*butoxycarbonyl)-2-methylalaninate (27)**. The compound 5-(acetyloxy)-2,2-dimethyl-3,4-dihydro-2*H-*benzo[*h*]chromen-6-yl *N-*(*tert-*butoxycarbonyl)-2-methylataninate (**27**) is synthesized as described in scheme 1a using zinc dust (2.0 g, 30.5 mmol), 2,2-dimethyl-3,4-dihydro-2*H*benzo[*h*]chromene-5,6-dione (2.0 g, 8.26 mmol), Na₂S₂O₄ (5.75 g, 33.0 mmol), *N-*(*tert-*butoxycarbonyl)-2-methylalanine (3.36 g, 16.51 mmol), triethylamine (1.04 mL, 7.43 mmol), HBTU (6.26 g, 16.5 mmol) and DMF (30 mL) for the 1^{st} step. The acetylation step is carried out using zinc dust (1.0 g, 15.3 mmol), thriethylamine (1.04 mL, 7.43 mmol) and acetic anhydride (20 mL). Both the compounds are generated in the reaction. The crude mixture is purified by two consecutive flash column chromatography (SiO₂, 1^{st} using a gradient from 100% dichloromethane to 2% EtOAc in dichloromethane and 2^{nd} using a gradient from 10% EtOAc in hexanes to 25% EtOAc in hexanes) to afford pure desired single isomer product (0.405 g, %) as a white solid. 400 MHz ¹H NMR (CDCl₃) δ: 8.19 (d, *J* = 8.8 Hz, 1H), 7.85 (d, *J* = 7.6 Hz, 1H), 7.50-7.35 (m, 2H), 5.1.9 (br. s, 1H), 2.67 (t, *J*= 6.6 Hz, 2H), 2.37 (s, 3H), 1.87 (t, *J*= 6.8 Hz, 2H), 1.78 (s, 6H), 1.47 (s, 9H), 1.42 (s, 6H); LCMS: 472 [M+H].

### Q Comparative Example 12: Synthesis of 5-(acetyloxy)-2,2-dimethyl-3,4-dihydro-2H-benzo[h]chromen-6-yl-2-methylalaninate hydrochloride (28)

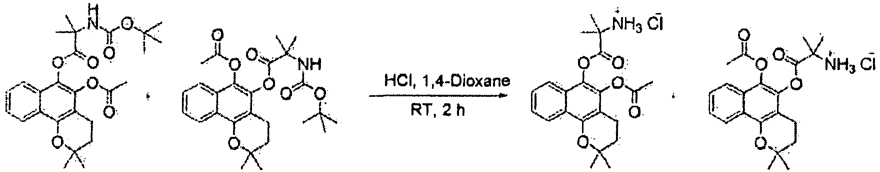

**5-(acetyloxy)-2,2-dimethyl-3,4-dihydro-2*H-*benzo[*h*]chromen-6-yl 2-methylalaninate hydrochloride (28)**. To a solution of 5-(acetyloxy)-2,2-dimethyl-3,4-dihydro-2*H-*benzo[*h*]chromen-6-yl *N-*(*tert-*butoxycarbonyl)-2-methylalaninate (**27**) (0.4 g, 0.85 mmol) in 1,4-dioxane (5 mL) is added a solution of hydrogen chloride gas in anhydrous 1,4-dioxane (4.0 M, 10 mL). The reaction is stirred at room temperature for 2 hours. The reaction is dried under reduced pressure and the resulting solid is triturated with Et₂O to afford the desired product as a white solid (0.388 g, 97%). M.p. = 283-284 °C; 400 MHz ¹H NMR (DMSO-d₆) δ: 8.93 (br, s, 3H), 8.15 (d, *J*= 8.4 Hz, 1 H), 7,72 (d, *J* = 8.0 Hz, 1H), 7.65-7.50 (m, 2H), 2.63 (t, *J* = 6.6 Hz, 2H), 2.38 (s, 3H), 1.89 (t, *J* = 6.6 Hz, 2H), 1.78 (s, 6H), 1.39 (s, 6H); LCMS: 372 [M+H]; Calc. for C₂₁H₂₅NO₅, 1.15 HCl: C 60.96, H 6.38, N 3.39; Found C 61.01, H 6.06, N 3.30.

### R. Comparative Example 13: Synthesis of 5-(acetyloxy)-2,2-dimethyl-3,4-dihydro-2H-benzo[h]chromen-6-yl N-(tert-butoxycarbonyl)-β-alaninate (29) and 6-(acetyloxy)-2,2-dimethyl-3,4-dihydro-2H-benzo[h]chromen-5-yl N-(tert-butoxycarbonyl)-β-alaninate (30)

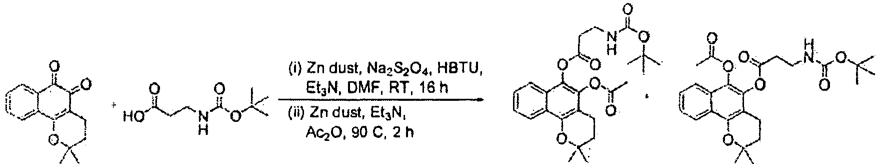

**5-(acetyloxy)-2,2-dimethyl-3,4-dihydro-2*H*-benzo[*h*]chromen-6-yl *N*-(*tert-*butoxycarbonyl)-β-alaninate (29) and 6-(acetyloxy)-2,2-dimethyl-3,4-dihydro-2*H-*benzo[*h*]chromen-5-yl *N*-(*tert-*butoxycarbonyl)-β-alaninate (30)**. The compounds 5-(acetyloxy)-2,2-dimethyl-3,4-dihydro-2*H*-benzo[*h*]chromen-6-yl *N*-(*tert-*butoxycarbonyl)-β-alaninate (**29**) and 6-(acetyloxy)-2,2-dimethyl-3,4-dihydro-2*H-*benzo[*h*]chromen-5-yl *N-*(*tert-*butoxycarbonyl)-β-alaninate (**30**) are synthesized as described in example 1. using zinc dust (2.0 g, 30.6 mmol),-2,2-dimethyl-3,4-dihydro-2*H-*benzo[*h*]chromene-5,6-dione (2.0 g, 8.26 mmol), Na₂S₂O₄ (5.75 g, 33.0 mmol), *N*-(*tert-*butoxycarbonyl)-β-alanine (3.12 g, 16.5 mmol), triethylamine (1.0 mL, 7.1 mmol), HBTU (6.2 g, 16.5 mmol) and DMF (30 mL) for the 1^{st} step. The acetylation step is carried out using zinc dust (2.0 g, 30.6 mmol), thriethylamine (2.0 mL, 14.2 mmol) and acetic anhydride (30 mL). Both the compounds are generated in the reaction. The crude mixture is purified by three consecutive flash column chromatography on SiO₂ (twice using a gradient from 10% EtOAc in hexanes to 25% EtOAc in hexanes once using 100% dichloromethane) to afford pure desired product as mixture of isomers (**29:30**) in a ratio of 7:1 as established by ¹H NMR. M.p. = 171-172 °C; 400 MHz ¹H NMR (CDCl₃) δ: 8.21 (d, *J* = 8.8 Hz, 1H), 7.64 (d, *J* = 8.0 Hz, 1H), 7.50-7.40 (m, 2H), 5.12 (br. s, 1H), 3.60-3.50 (m, 2H), 2.94 (t, *J*= 6.0 Hz, 2H), 2.69 (t, *J*= 6.6 Hz, 2.H), 2.35 (s, 3H), 1.88 (t, *J* = 6.8 Hz, 2H), 1.46 (s, 9H), 1.43 (s, 6H); LCMS: 458 [M+H]; Calc. for C₂₅H₃₁NO₇: C 65.57, H 6.83, N 3.06; Found C 65.69, H 6.23, N 3.19.

### S. Example 6: Synthesis of 5-(acetvloxy)-2,2-dimethyl-3,4-dihydro-2H-benzo[h]chromen-6-yl-β-alaninate hydrochloride (31) and 6-(acetyloxy)-2,2-dimethyl-3,4-dihydro-2H-benzo[h]chromen-5-yl-β-alaninate hydrochloride (32)

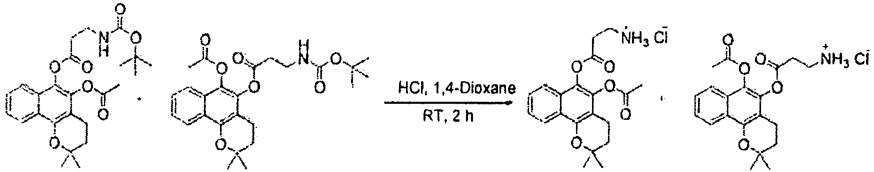

5-(acetyloxy)-2,2-dimethyl-3,4-dihydro-2*H-*benzo[*h*]chromen-6-yl-β-alaninate hydrochloride (31 - Prodrug 8) and 6-(acetyloxy)-2,2-dimethyl-3,4-dihydro-2*H*-benzo[*h*]chromen-5-yl-β-alaninate hydrochloride (32). To a solution of 5-(acetyloxy)-2,2-dimethyl-3,4-dihydro-2H-benzo[h]chromen-6-yl *N*-(*tert-*butoxycarbonyl)-β-alaninate (29) and 6-(acetyloxy)-2,2-dimethyl-3,4-dihydro-2H-benzo[h]chromen-5-yl *N-*(*tert-*butoxycarbonyl)-β-alaninate (30) (1.0 g, 2.18 mmol) in 1,4-dioxane (5 mL) is added a solution of hydrogen chloride gas in anhydrous 1,4-dioxane (4.0 M; 5 mL). The reaction is stirred at room temperature for 16 hours. The reaction is dried under reduced pressure. The product is obtained as a white solid (0.55 g, 64%) after recrystallization in 30 of toluene and is a mixture of both the isomers 5-(acetyloxy)-2,2-dimethyl-3,4-dihydro-2*H*-benzo[*h*]chromen-6-yl-β-alaninate hydrochloride (**31**) and 6-(acetyloxy)-2,2-dimethyl-3,4-dihydro-2*H*-benzo[*h*]chromen-5-yl-β-alaninate hydrochloride (**32**). The ratio of the isomers **31:32** is determined to be 10:1 by ¹H NMR. M.p. = 1.73-176 °C; 400 MHz ¹H NMR (DMSO-d₆) δ: 8.20-8.05 (m, 4H), 7.84 (d, *J*= 7.6 Hz, 1H), 7.60-7.45 (m, 2H), 3.25-3.15 (m, 4H), 2.62 (t,. *J*= 6.6 Hz, 2H), 2.40 (s, 3H), 1.87 (t, *J* = 6.6 Hz, 2H), 1.39 (s, 6H); LCMS: 387 [M+H]; Calc. for C₂₀H₂₃NO₅ 1.38 HCl: C 58.86, H 6.03, N 3.43; Found C 58.94, H 5.67, N 3.62_{.}

### T. Example 7: Synthesis of 5-Acetoxy-2,2-dimethyl-3.4-dihydro-2H-benzo[h]chromen-6-yloxycarbonylmethyl-ammonium; chloride

**5-Acetoxy-2,2-dimethyl-3,4-dihydro-2*H*-benzo[*h*]chromen-6-yloxycarbonylmethyl-ammonium; chloride.** A preferred β-lapachone prodrug composition of the invention using glycine as the amino acid moiety may be prepared in general, accordance with Scheme la as follows.

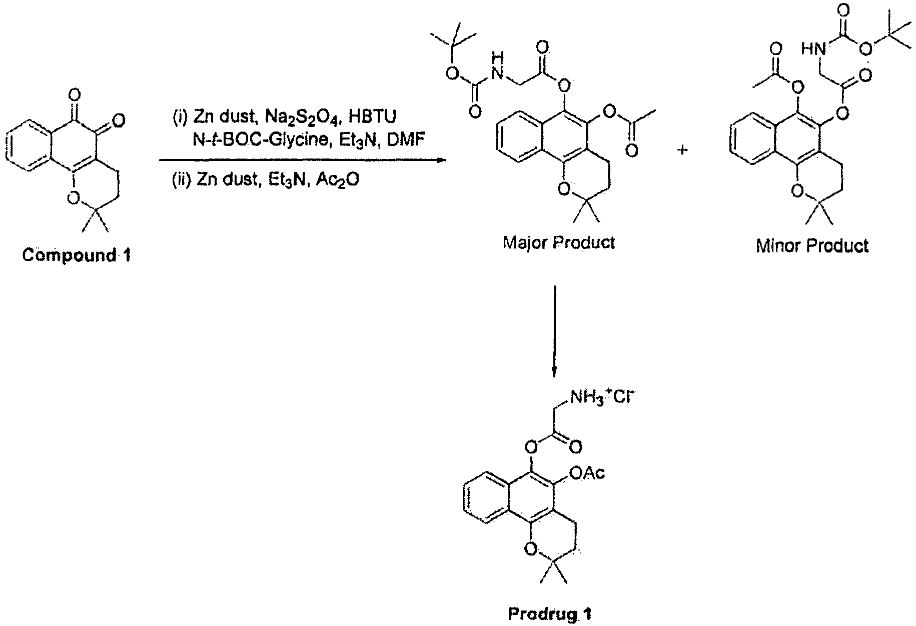

### U. Comparative Example 14: Stability of_β-Lapachone Prodrug Composition and Release of β-Lapachone

The release of β-lapachone from a β-lapachone prodrug composition of the invention may be demonstrated under basic conditions (1.0 N NaOH). For example, Figure 1 shows the release of β-lapachone from Prodrug 1 at varying pH values.

## Claims

1. A quinone prodrug composition comprising a quinone compound of formula Ia: 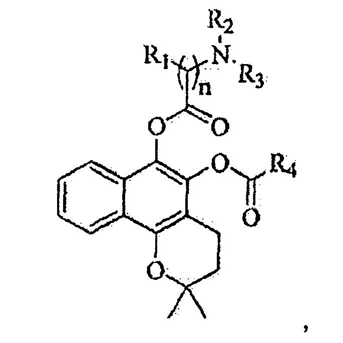 wherein:
R₁ is H, alkyl, alkyl substituted with a sulfyl (-SH or thio alkyl), or (C₁-C₄) alcohol, or together with R₂, forms a saturate, unsaturated, or aromatic cyclic moiety;
R₂ and R₃ are each independently H, alkyl, or aromatic, or together form a saturated, unsaturated, or aromatic cyclic moiety
R₄ is alkyl, the side chain of a naturally occurring alpha amino acid, or the side chain, alpha carbon, and the alpha amino group of a naturally occurring amino acid; and
n is 1, 2 or 3.

2. The quinone prodrug composition of claim 1, wherein the quinone compound is selected from the group consisting of: 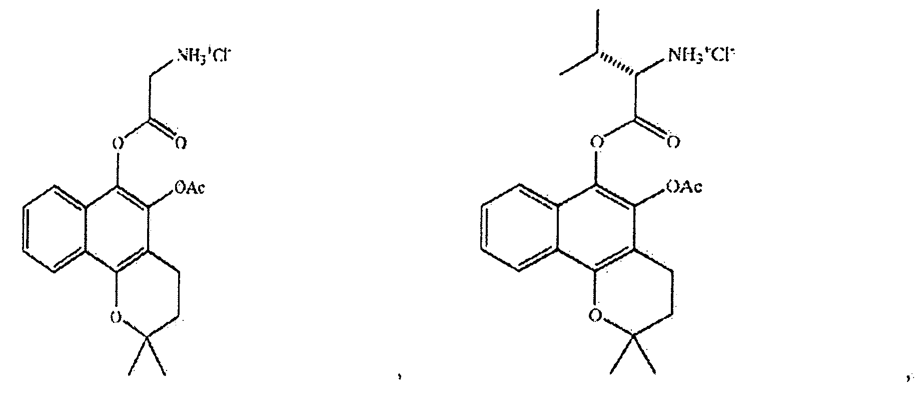 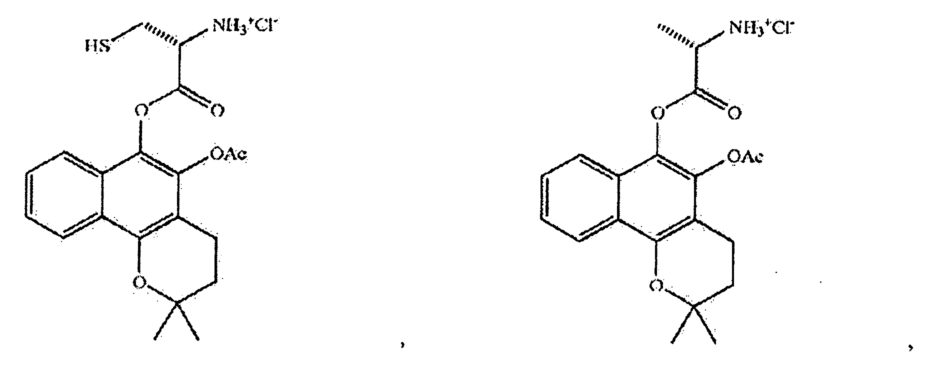 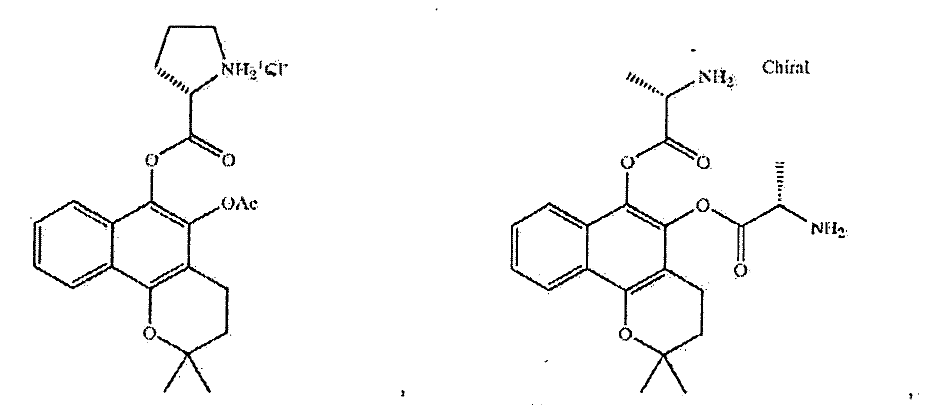 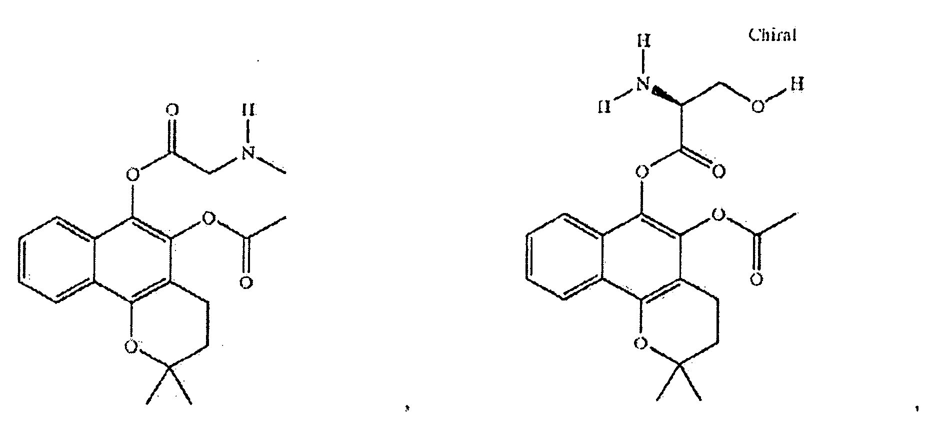 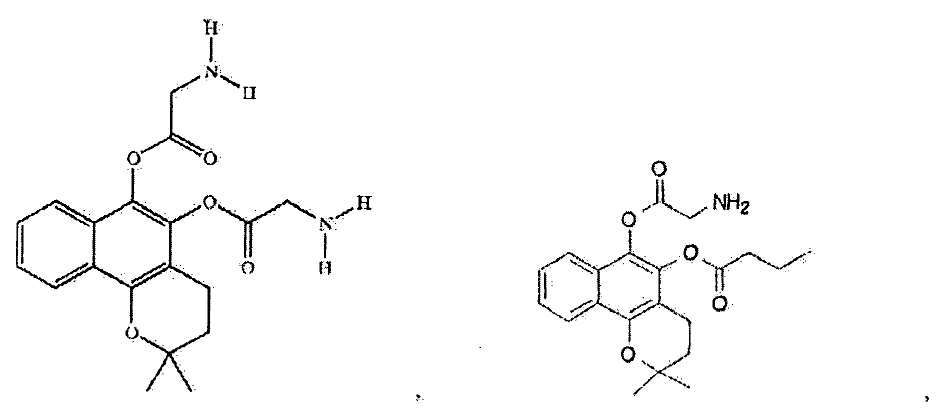 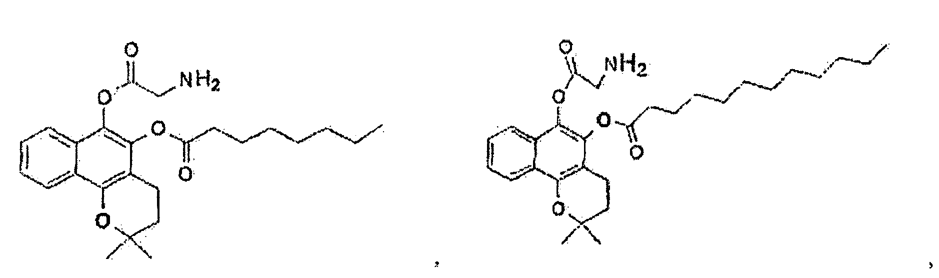 and

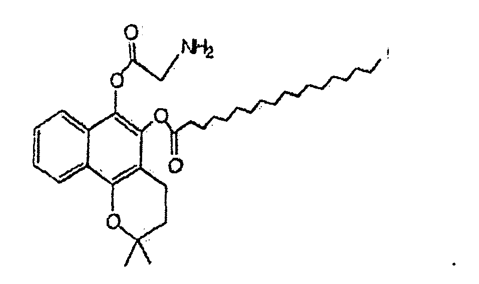

3. The quinone prodrug composition of claim 1, wherein the quinone compound is: 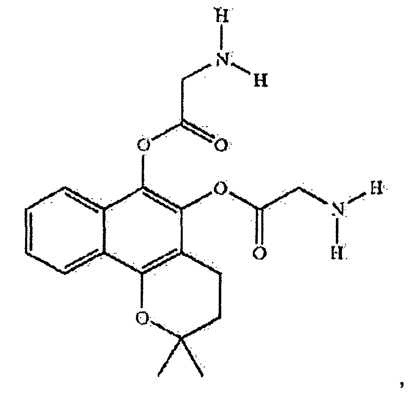 or a HCl salt, a mesylate salt or a tosylate salt thereof.

4. A pharmaceutical composition comprising a therapeutically effective amount of at least one quinone prodrug composition of claim 1 and a pharmaceutically acceptable excipient.

5. The pharmaceutical composition of claim 4, wherein the pharmaceutical composition is an aqueous solution.

6. The pharmaceutical composition of claim 4, wherein the pharmaceutical composition is a lyophilized solid.

7. The pharmaceutical composition of claim 4, wherein the pharmaceutical composition comprises 0.1 mg/ml to 10 mg/ml of the quinone prodrug composition.

8. The pharmaceutical composition of claim 4, further comprising a second anticancer agent.

9. The pharmaceutical composition of claim 8, wherein the second anticancer agent is selected from the group consisting of taxane derivatives, gemcitabine, nucleoside and nucleotide anticancer agents, cisplatin, imatnibmeasylafe, and trastuzumab.

10. The pharmaceutical composition of claim 9, wherein the taxane derivative is paclitaxel or docetaxol.

## Patentansprüche

1. Chinon-Pro-Pharmakon-Zusammensetzung, die eine Chinonverbindung der Formel la umfasst: 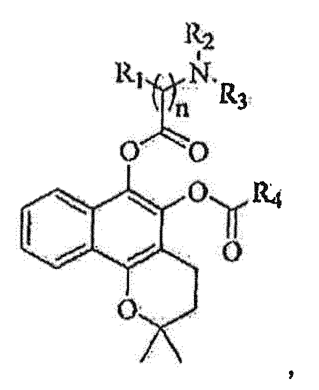 worin:
R₁ H, Alkyl, Alkyl, das mit einer Sulfylgruppe (-SH oder Thioalkyl) substituiert ist, oder ein (C₁-C₄)-Alkohol ist oder zusammen mit R₂ einen gesättigten, ungesättigten oder aromatischen cyclischen Rest bildet,
R₂ und R₃ unabhängig H, Alkyl oder ein aromatischer Rest sind oder zusammen einen gesättigten, ungesättigten oder aromatischen cyclischen Rest bilden,
R₄ Alkyl, die Seitenkette einer natürlich vorkommenden alpha-Aminosäure oder die Seitenkette, das alpha-Kohlenstoffatom und die alpha-Aminogruppe einer natürlich vorkommenden Aminosäure ist, und
n 1, 2 oder 3 ist.

2. Chinon-Pro-Pharmakon-Zusammensetzung nach Anspruch 1, wobei die Chinonverbindung aus der Gruppe, bestehend aus 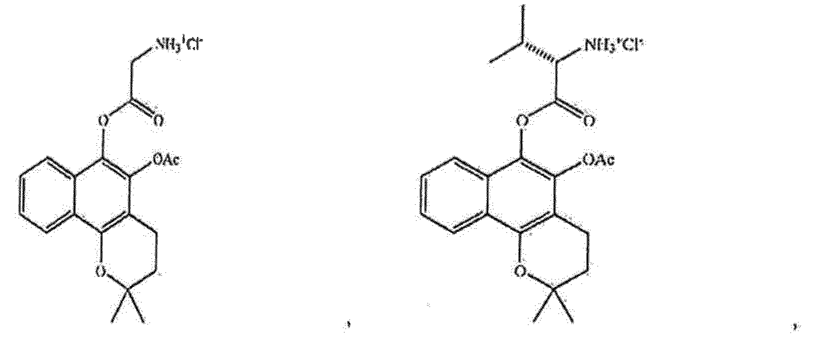 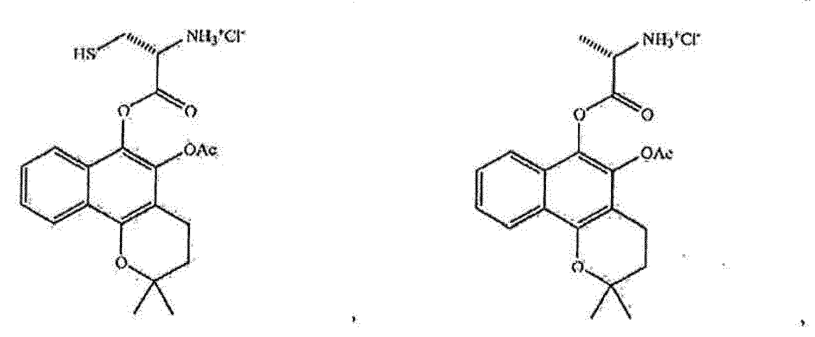 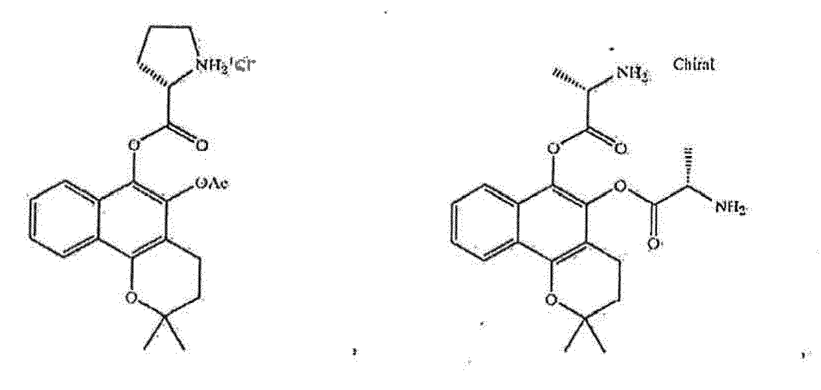 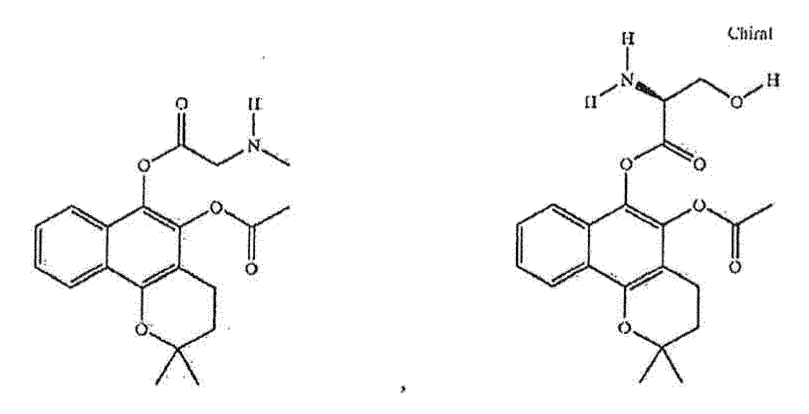 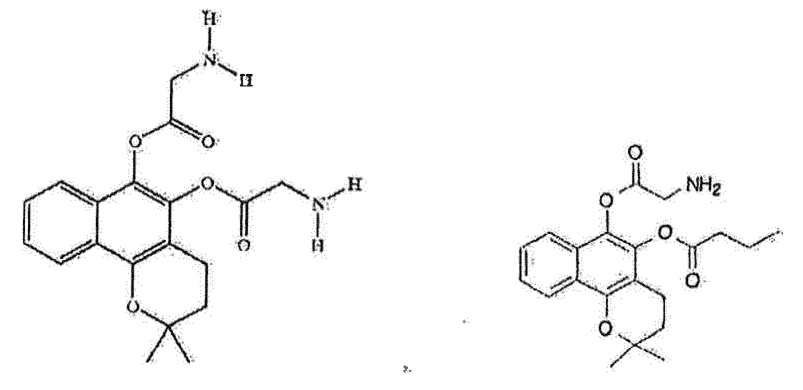 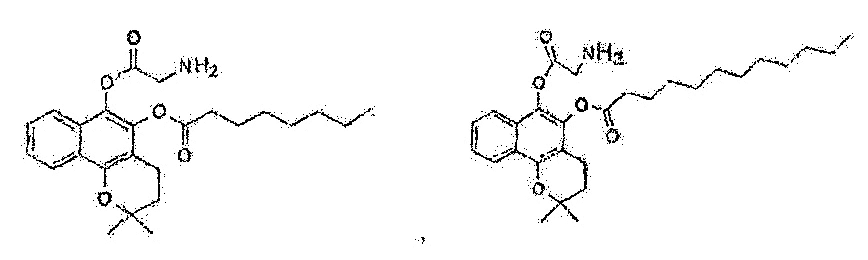 und 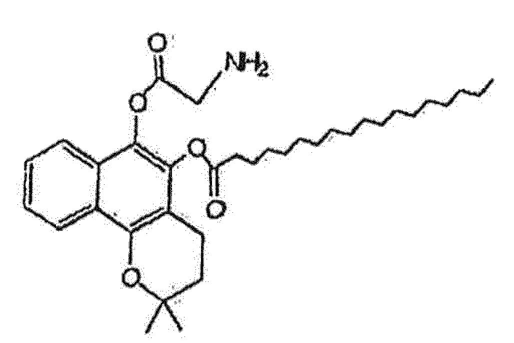 ausgewählt ist.

3. Chinon-Pro-Pharmakon-Zusammensetzung nach Anspruch 1, wobei die Chinonverbindung 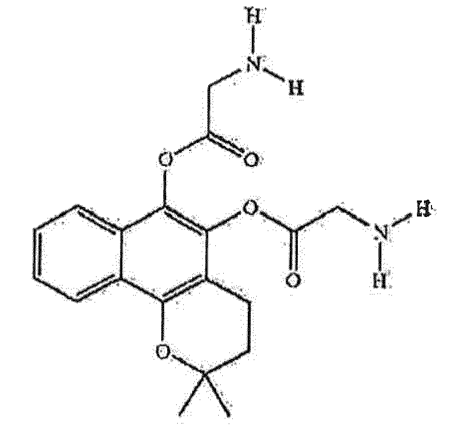 oder ein HCl-Salz, ein Mesylatsalz oder ein Tosylatsalz davon ist.

4. Pharmazeutische Zusammensetzung, die eine therapeutisch wirksame Menge von mindestens einer Chinon-Pro-Pharmakon-Zusammensetzung nach Anspruch 1 und einen pharmazeutisch verträglichen Träger umfasst.

5. Pharmazeutische Zusammensetzung nach Anspruch 4, wobei die pharmazeutische Zusammensetzung eine wässrige Lösung ist.

6. Pharmazeutische Zusammensetzung nach Anspruch 4, wobei die pharmazeutische Zusammensetzung ein lyophilisierter Feststoff ist.

7. Pharmazeutische Zusammensetzung nach Anspruch 4, wobei die pharmazeutische Zusammensetzung 0,1 mg/ml bis 10 mg/ml der Chinon-Pro-Pharmakon-Zusammensetzung umfasst.

8. Pharmazeutische Zusammensetzung nach Anspruch 4, die ferner ein zweites Antikrebsmittel umfasst.

9. Pharmazeutische Zusammensetzung nach Anspruch 8, wobei das zweite Antikrebsmittel aus der Gruppe, bestehend aus Taxanderivaten, Gemcitabin, Nukleosid- und ukleotid-Antikrebsmitteln, Cisplatin, Imatinibmesylat und Trastuzumab, ausgewählt ist.

10. Pharmazeutische Zusammensetzung nach Anspruch 9, wobei das Taxanderivat Paclitaxel oder Docetaxol ist.

## Revendications

1. Composition de promédicament de quinone comprenant un composé quinone de formule Ia : 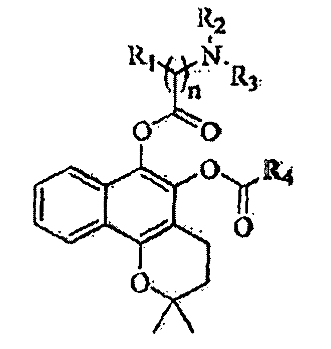 dans laquelle :
R₁ est H, alkyle, alkyle substitué par un sulfyle (-SH ou thioalkyle), ou un alcool en C₁ à C₄, ou bien, conjointement avec R₂, forme un fragment cyclique saturé, insaturé ou aromatique ;
chacun de R₂ et R₃ est indépendamment H, alkyle ou aromatique, ou bien ils forment ensemble un fragment cyclique saturé, insaturé ou aromatique ;
R₄ est alkyle, la chaîne latérale d'un acide α-aminé naturel, ou la chaîne latérale, α-carbone, et le groupe α-amino d'un acide amine naturel ; et
n vaut 1, 2 ou 3.

2. Composition de promédicament de quinone selon la revendication 1, dans laquelle le composé quinone est choisi dans l'ensemble comprenant les suivants : 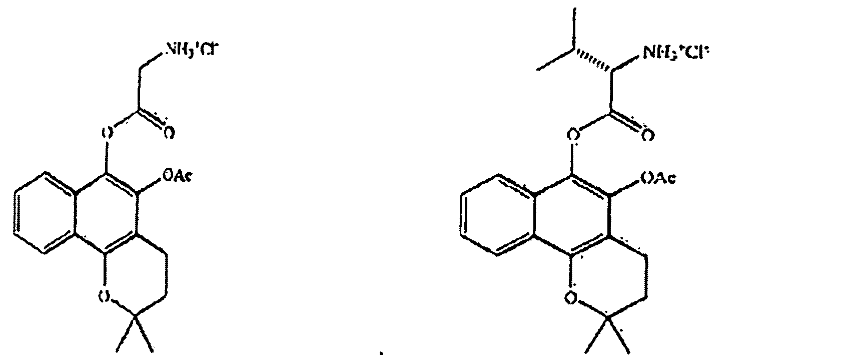 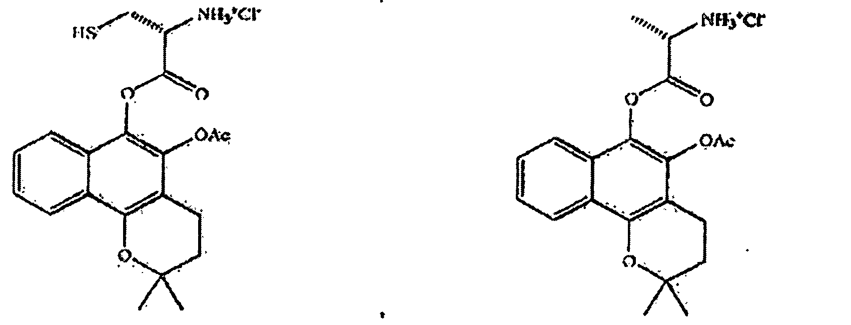 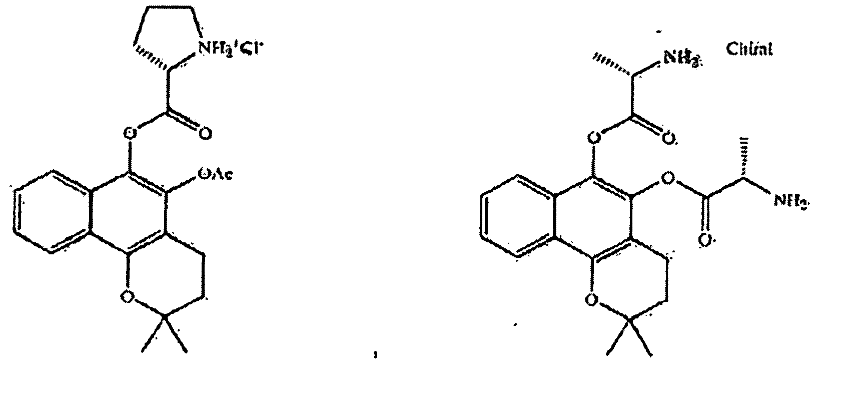 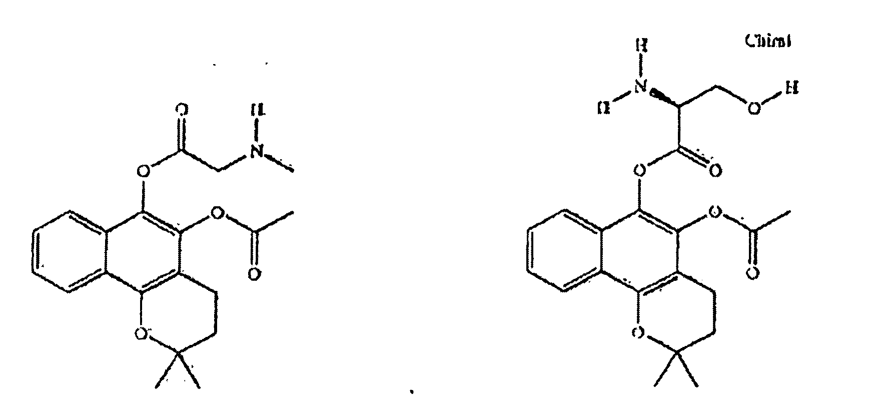 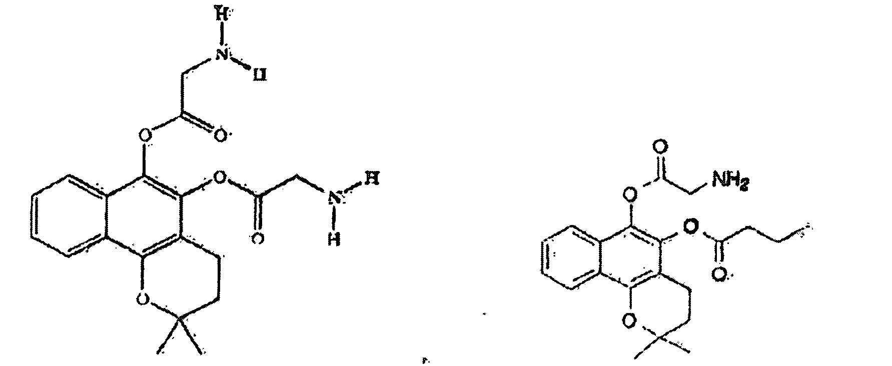 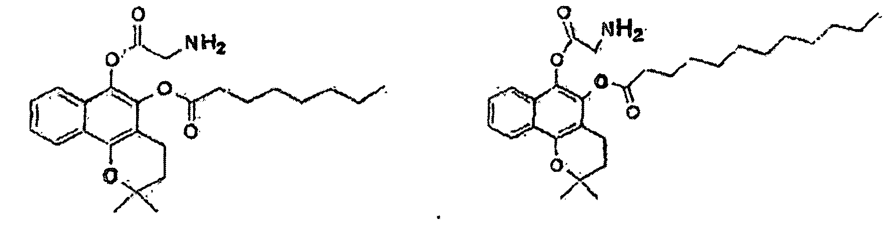 et

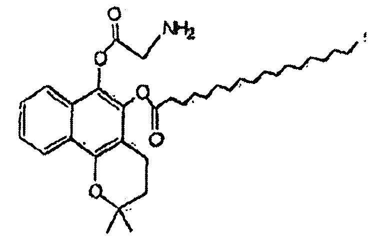

3. Composition de promédicament de quinone selon la revendication 1, dans laquelle le composé quinone est : 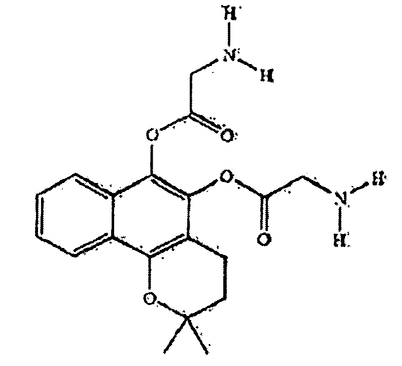 ou un sel de HCl, un sel mésylate ou un sel tosylate de celui-ci.

4. Composition pharmaceutique comprenant une quantité efficace, du point de vue thérapeutique, d'au moins une composition de promédicament de quinone selon la revendication 1 et un excipient pharmaceutiquement acceptable.

5. Composition pharmaceutique selon la revendication 4, laquelle composition pharmaceutique est une solution aqueuse.

6. Composition pharmaceutique selon la revendication 4, laquelle composition pharmaceutique est un solide lyophilisé.

7. Composition pharmaceutique selon la revendication 4, laquelle composition pharmaceutique comprend de 0,1 mg/ml à 10 mg/ml de la composition de promédicament de quinone.

8. Composition pharmaceutique selon la revendication 4, comprenant en outre un deuxième agent anticancéreux.

9. Composition pharmaceutique selon la revendication 8, dans laquelle le deuxième agent anticancéreux est choisi dans l'ensemble constitué par les dérivés de taxane, la gemcitabine, les agents anticancéreux nucléosidiques et nucléotidiques, le cisplatine, le mésylate d'imatinib, et le trastuzumab.

10. Composition pharmaceutique selon la revendication 9, dans laquelle le dérivé de taxane est le paclitaxel ou lie docétaxol.
